(19) Europäisches Patentamt
European Patent Office
Office européen des brevets

(11) Publication number: 0 256 888 B1

# (12) EUROPEAN PATENT SPECIFICATION

(45) Date of publication of patent specification: 03.07.91

(51) Int. Cl.5: C07D 281/10, C07D 417/12, A61K 31/55

(21) Application number: 87307316.7

(22) Date of filing: 19.08.87

## (54) Benzothiazepine vasodilators having aralkyl substitution.

(30) Priority: 20.08.86 US 898298

(43) Date of publication of application:
24.02.88 Bulletin 88/08

(45) Publication of the grant of the patent:
03.07.91 Bulletin 91/27

(84) Designated Contracting States:
AT BE CH DE ES FR GB GR IT LI LU NL SE

(56) References cited:
EP-A- 0 127 882
EP-A- 0 154 838
EP-A- 0 182 273

(73) Proprietor: McNeilab, Inc.
Spring House Pennsylvania(US)

(72) Inventor: Carson, John Robert
551 Rittenhouse Blvd.
Norristown, PA 19403(US)

(74) Representative: Jones, Alan John et al
CARPMAELS & RANSFORD 43 Bloomsbury Square
London, WC1A 2RA(GB)

EP 0 256 888 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Background of the Invention

Diltiazem having a chemical name of 3-(acetyloxy)-5-[2-(dimethylamino)ethyl]-2,3-dihydro-2-(4-methoxyphenyl)-1,5-benzothiazepin-4(5H)-one is a coronary vasodilator useful in the treatment of angina and has calcium blocking activity as described in U.S. Patent 3,562,257. In addition, it has recently been reported by Robert S. Gibson et al. in the New England Journal of Medicine, Vol. 315, Issue #7, pages 423-429 (August 14, 1986) that diltiazem prevents an often fatal second heart attack from occurring during a patient's recovery from a first heart attack. Other 1,5-benzothiazepines are disclosed in U.S. Patent 4,585,768 and in published European Patent Application 158,340.

Other calcium blockers useful against angina are those described in U.S. Serial No. 665,684 filed October 29, 1984 which corresponds to published European Patent Application 146,271 published June 26, 1985.

It is an object of the present invention to provide calcium blockers having high levels of activity in the treatment of angina pectoris in mammals, e.g. humans, and having activity in the prevention of heart attacks in mammals, particularly the occurrence of a second heart attack by treatment of a patient during recovery from a first heart attack.

### Summary of the Invention

Novel benzothiazepines of the following formula (I):

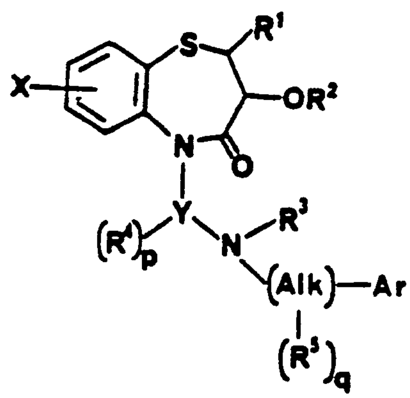

(I)

have been found to be extremely potent vasodilators as seen in the Langendorff isolated heart screen. As such, they would be considered to be useful as agents in the treatment of angina. The invention compounds may also be used in the treatment of hypertension and prevention of the reoccurrence of heart attacks in humans.

### Detailed Description of the Invention

Benzothiazepines of the following formula (I):

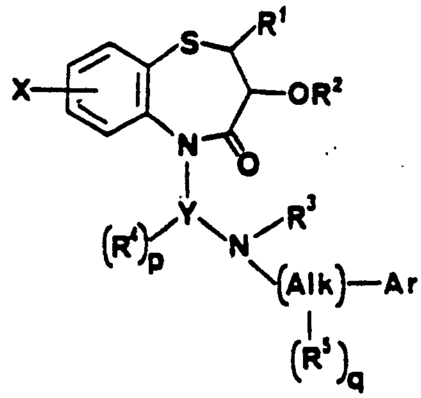

(I)

wherein

X is a hydrogen atom or a halogen atom;

$R^1$ is phenyl or phenyl independently substituted by 1 to 3 of methyl, ethyl, n-propyl, isopropyl, n-butyl, sec-butyl, tert-butyl, iso-butyl, methoxy, ethoxy, n-propoxy, iso-propoxy, n-butoxy, sec-butoxy, tert-butoxy, iso-butoxy, or halogen;

$R^2$ is hydrogen, acetyl, propionyl, 2-methyl-propionyl or butyryl;

Y is straight chain alkylene of 2 or 3 carbons;

$R^3$ is methyl, ethyl, n-propyl, iso-propyl, n-butyl, sec-butyl or iso-butyl;

$R^4$ is independently methyl, ethyl, n-propyl, iso-propyl, n-butyl, sec-butyl or iso-butyl;

p is 0, 1, 2 or 3;

Alk is ethylene, trimethylene or tetramethylene;

$R^5$ is hydroxy, alkyl of 1 to 4 carbons or phenyl;

q is 0, 1, 2 or 3; and

Ar is phenyl, phenoxy, thiophenoxy, naphthyl or a 5- or 6-membered heterocyclic aromatic ring having one nitrogen, sulfur or oxygen atom which rings may be substituted independently by one or more of alkyl, alkoxy or alkylthio of 1 to 6 carbons each, hydroxy, halogen, fluoroalkyl of 1 to 6 carbons, nitro, amino, dialkylamino of 2 to 12 carbons or methylenedioxy at adjacent ring cabons,

and the pharmaceutically acceptable acid addition salts and quaternary ammonium compounds thereof.

X in more detail is hydrogen, fluoro, chloro, bromo or iodo.

Examples of $Y(R^4)_p$ are $-CH_2CH_2-$, $-CH(CH_3)CH_2-$ and $-CH_2CH(CH_3)-$.

Examples of Ar are 1- or 2-naphthyl, furan or thiophene attached at the 2 or 3 position, pyrrole attached at the 1, 2 or 3 position and pyridine attached at the 2, 3 or 4 position. The open positions of the ring, or rings in the case of naphthyl, of Ar may be substituted by one or more, e.g. one or two, same or different, of alkyl of 1 to 6 carbons such as methyl or ethyl; alkoxy of 1 to 6 carbons such as methoxy and ethoxy; alkylthio of 1 to 6 carbons such as methylthio; hydroxy; halogen such as fluoro, chloro and bromo; fluoroalkyl of 1 to 6 carbons and one or more fluorine atoms with examples being 2,2,2-trifluoroethyl and trifluoromethyl; nitro; amino; or dialkylamino of 2 to 12 carbons such as dimethylamino; or methylenedioxy at adjacent ring carbons particularly if Ar is phenyl, phenoxy or thiophenoxy; e.g. 3,4-methylenedioxyphenyl. Preferably in formula (I), the hydroxy for $R^5$ is not attached to the same carbon as the nitrogen atom in formula (I).

Particular embodiments of the present invention include compounds of formula (I) wherein

X is hydrogen;

$R^1$ is phenyl substituted by lower alkoxy;

$R^2$ is lower alkanoxy;

Y is ethylene;

$R^3$ is lower alkyl;

p is 0;

Alk is ethylene;

q is 0; and

Ar is phenyl or phenyl substituted independently by 1 or 2 of alkyl, alkoxy, alkylthio, halogen, fluoroalkyl, amino or dialkylamino or by methylenedioxy at adjacent ring carbons.

Most particularly, Ar is phenyl independently substituted by 1 or 2 of methyl, ethyl, n-propyl, iso-propyl, methoxy, ethoxy, n-propyl, iso-propoxy, fluoro, chloro, bromo or iodo. Specific examples include 3-methoxyphenyl, 3,4-dimethoxyphenyl, 3,4-dichlorophenyl, 3,4-methylenedioxyphenyl, 3-methylphenyl, 2-methoxyphenyl, 3,5-dimethoxyphenyl and 2,3-dichlorophenyl.

Particular compounds of the inventions are:

3-(Acetyloxy)-5-[2-[[2-(3-methoxyphenyl)ethyl]methylamino]ethyl]-2,3-dihydro-2-(4-methoxyphenyl)-1,5-benzothiazepine-4(5H)-one;

3-Hydroxy-5-[2-[[2-(3-methoxyphenyl)ethyl]methylamino]ethyl]2,3-dihydro-2-(4-methoxyphenyl)-1,5-benzothiazepine-4(5H)-one;

3-(Acetyloxy)-5-[2-[[2-(3,4-dimethoxyphenyl)ethyl]methylamino]ethyl]-2,3-dihydro-2-(4-methoxyphenyl)-1,5-benzothiazepine-4(5H)-one;

3-(Hydroxy)-5-[2-[[2-(3,4-dimethoxyphenyl)ethyl]methylamino]ethyl]-2,3-dihydro-2-(4-methoxyphenyl)-1,5-benzothiazepine-4(5H)-one;

3-(Acetyloxy)-5-[2-[[2-(3,4-dichlorophenyl)ethyl]methylamino]ethyl]-2,3-dihydro-2-(4-methoxyphenyl)-1,5-benzothiazepine-4(5H)-one;

3-(Hydroxy)-5-[2-[[3,4-dichlorophenyl)ethyl]methylamino]ethyl]-2,3-dihydro-2-(4-methoxyphenyl)-1,5-benzothiazepine-4(5H)-one;

3-(Acetyloxy)-5-[2-[[2-(3,4-methylenedioxyphenyl)ethyl]methylamino]ethyl]-2,3-dihydro-2-(4-methoxyphenyl)-1,5-benzothiazepine-4(5H)-one;

3-Hydroxy-5-[2-[[2-(3,4-methylenedioxyphenyl)ethyl]methylamino]ethyl]-2,3-dihydro-2-(4-methoxyphenyl)-1,5-benzothiazepine-4(5H)-one;

3-(Acetyloxy)-5-[2-[[2-(3-methylphenyl)ethyl]methylamino]ethyl]-2,3-dihydro-2-(4-methoxyphenyl)-1,5-benzothiazepine-4(5H)-one;

3-(Acetyloxy)-5-[2-[[2-methoxyphenyl)ethyl]methylamino]ethyl]-2,3-dihydro-2-(4-methoxyphenyl)1,5-benzothiazepine-4(5H)-one;

3-(Acetyloxy)-5-[2-[[2-(3,5-dimethoxyphenyl)ethyl]methylamino]ethyl]-2,3-dihydro-2-(4-methoxyphenyl)-1,5-benzothiazepine-4(5H)-one;

3-(Acetyloxy)-5-[2-[[2,3-dichlorophenyl)ethyl]methylamino]ethyl]-2,3-dihydro-2-(4-methoxyphenyl)-1,5-benzothiazepine-4(5H)-one;

3-(Acetyloxy)-5-[(S)-2-[[2-(3,4-dimethoxyphenyl)ethyl]methylamino]propyl]-2,3-dihydro-2-(4-methoxyphenyl)-1,5-benzothiazepin-4(5H)-one;

3-(Acetyloxy)-5-[(S)-2-[[2-(3,4-dimethoxyphenyl)ethyl]methylamino]-1-methylethyl]-2,3-dihydro-2-(4-methoxyphenyl)-1,5-benzothiazepin-4(5H)-one;

3-(Acetoxy)-5-[(S)-2-[[2-(2,3-dichlorophenyl)ethyl]methylamino]propyl]-2,3-dihydro-2-(4-methoxyphenyl)-1,5-benzothiazepin-4(5H)-one

cis-3-(acetyloxy)-5-[2-[[2-(3,4-dimethoxyphenyl)ethyl]methylamino]ethyl]-2,3-dihydro-2-(4-methoxyphenyl)-1,5-benzothiazepine-4(5H)-one;

cis-3-(acetyloxy)-5-[2-[[2-(3,5-dimethoxyphenyl)ethyl]methylamino]ethyl]-2,3-dihydro-2-(4-methoxyphenyl)-1,5-benzothiazepine-4(5H)-one;

cis-3-(acetyloxy)-5-[2-[[2-(3,4-dichlorophenyl)ethyl]-methylamino]ethyl]-2,3-dihydro-2-(4-methoxyphenyl)-1,5-benzothiazepine-4(5H)-one;

cis-3-(acetyloxy)-5-[2-[[2-(2,3-dichlorophenyl)ethyl]-methylamino]ethyl]-2,3-dihydro-2-(4-methoxyphenyl)-1,5-benzothiazepine-4(5H)-one;

cis-3-(acetyloxy)-5-[2-[[2-(3-methoxyphenyl)ethyl]-methylamino]ethyl]-2,3-dihydro-2-(4-methoxyphenyl)-1,5-benzothiazepine-4(5H)-one;

cis-3-(acetyloxy)-5-[2-[[2-(3-dimethylaminophenyl)ethyl]methylamino]ethyl]-2,3-dihydro-2-(4-methoxyphenyl)-1,5-benzothiazepine-4(5H)-one;

cis-3-(acetyloxy)-5-[2-[[2-(3-trifluoromethylphenyl)ethyl]methylamino]ethyl]-2,3-dihydro-2-(4-methoxyphenyl)-1,5-benzothiazepine-4(5H)-one;

cis-3-(acetyloxy)-5-[2-[[2-(3,4-dimethoxyphenyl)propyl]methylamino]ethyl]-2,3-dihydro-2-(4-methoxyphenyl)-1,5-benzothiazepine-4(5H)-one;

cis-5-[2-[[2-(3,4-Dimethoxyphenyl)ethyl]methylamino]ethyl]2,3-dihydro-3-hydroxy-2-(4-methoxyphenyl)-1,5-benzothiazepine-4(5H)-one; and

cis-3-(acetyloxy)-5-[2-[[2-(3,4-dimethoxyphenyl)ethyl]methylamino]propyl]-2,3-dihydro-2-(4-methoxyphenyl)-1,5-benzothiazepine-4(5H)-one.

Particular Alk($R^5$)$_q$Ar groups include 3,5-dimethoxyphenyl, 2,3-dichlorophenyl, 3,4-dichlorophenyl and 3-diethylaminophenyl. Particular compounds of the invention are the benzothiazepine of the following formula (VI) and (VII):

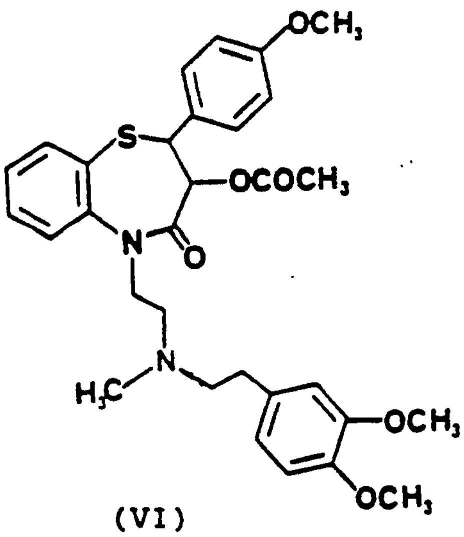

(VI)

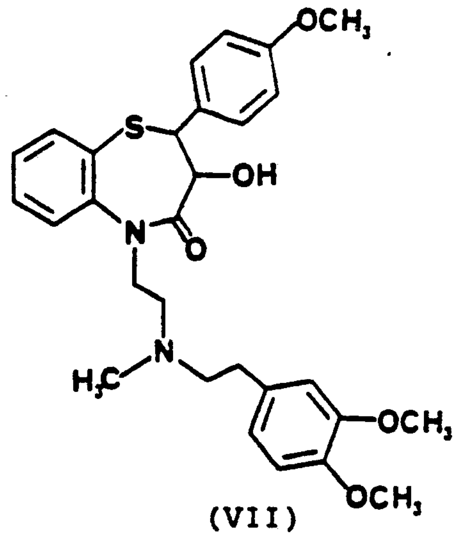

(VII)

wherein all substituents are as defined above for formula (I).

Salts, in particular, include those formed from acids such as hydrochloric, hydrobromic, hydroiodic, sulfuric, phosphoric, fumaric, maleic, cyclohexylsulfamic, citric, lactic and methanesulfonic and said quaternary ammonium compounds are those formed with an alkylhalide or alkylsulfate of about 1 to 6 carbons, e.g. an alkyl bromide such as methyl iodido. Compounds of formula (I) and other compounds of the invention may exist in various isomeric forms, e.g. in view of the presence of an asymmetric carbon or by being cis or trans at the 2- and 3-positions of the benzothiazepine ring. It is understood that the present invention includes all such individual isomers and their racemates. Particular asymmetric carbons are those at the 2- and 3-positions of the thiazepine 7-membered ring. The stereochemistry at these two positions may be cis or trans.

Compounds of the present invention may be prepared by either of the following two general Reaction Schemes.

<u>Reaction Scheme 1:</u>

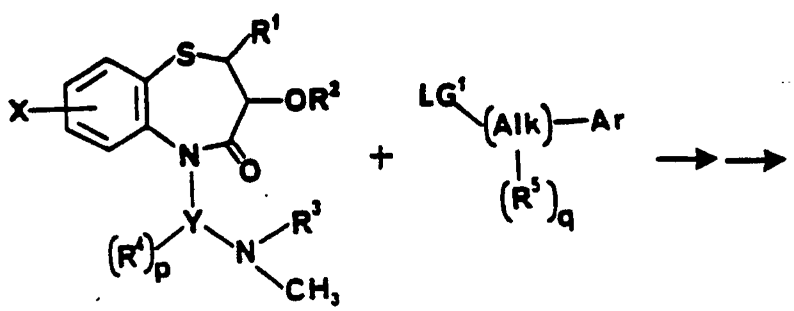

(I)

(II) (III)

In Reaction Scheme 1, a methylamino benzothiazepine (II) is reacted with an alkylating agent of the formula (III) wherein $LG^1$ is a leaving group such as tosyl, iodo, mesyl or bromo, with X, $R^1$, $R^2$, Y, $R^3$, $R^4$, p. Alk, Ar, $R^5$ and q all as described above for (I), at about room temperature up to 150 °C to produce the quaternary ammonium salt conjugate of (II) and (III). The intermediate salt may then be reacted without isolation with a strong nucleophile such as sodium thiophenoxide as described by T. Manoharan et al. in Synthesis, page 809-812 (1983) to yield the product of formula (I).

<u>Reaction Scheme 2:</u>

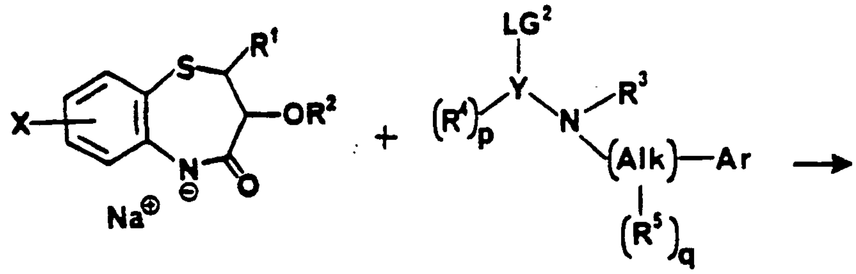

(I)

(IV) (V)

In Reaction Scheme 2, the salt (IV) is prepared as described in U.S. Patent 3,562,257 at column 3. The salt (IV) is then reacted with an alkylating agent of the formula (V), wherein $LG^2$ is a leaving group such as chlorine or bromine at a temperature of about 25 to 150° C to produce the benzothiazepine of formula (I). In formulae (IV) and (V), X, $R^1$, $R^2$, $R^3$, $R^4$, p, Y, Alk, Ar, $R^5$ and q are all as described for formula (I).

The compounds of formula (I) are useful in the treatment of the symptoms of angina pectoris by virtue of their ability to dilate coronary arteries. Their activity may be measured using the "Langendorff's isolated heart" preparation. This test has been described in "Pharmacological Experiments on Isolated Preparations". Staff of the Department of Pharmacology, University of Edinbourgh, 2nd Ed., Churchill Livingstone, N.Y., 1970, pp. 112-119. The test compounds may be administered at concentrations of 3000, 1000, 300, 100, 30, 10, 3, 1, 0.3, .1, 0.03 and 0.01 nanomolar ($10^{-9}$ molar).

In the Langendorff test, the known compound diltiazem was found to have a $C_{175}$ (the concentration needed to give a coronary flow of 175% of control) of 0.1µM. In contrast, the compound of the invention prepared in Example 4 had a $C_{175}$ of 0.000126µM.

The activity of compounds of formula (I) for the treatment of hypertension may be determined using the Spontaneously Hypertensive Rat (SHR) test as described below.

In this test, the arterial pressure of adult spontaneously hypertensive rats (Charles River) is monitored directly via an aortic cannula. The SH rats are anesthetized with an inhalation anesthetic (ether). The left carotid artery is isolated and cannulated. The tip of the cannula is advanced to the aorta and the cannula is exteriorized behind the neck at the level of the scapula. Animals are placed in individual cages and allowed to recover from the anesthetic and are kept unrestrained. The arterial cannula is connected to the pressure transducer which is attached to the recorder. The test compounds are administered to at least 3 rats at doses selected in the range of 0.1 to 100mg/kg of body weight by intraperitoneal (i.p.) or oral (p.o.) routes of administration. The arterial pressure and heart rate are monitored for a minimum of 24 hours. A test compound is considered to be active as an antihypertensive agent if the mean arterial pressure (MAP) indicates a fall of >15 mm of Hg. Each animal serves as its own control.

For the treatment of hypertension, angina or the prevention of heart attacks, compounds of the present invention of the formula (I) may be administered orally or parenterally in a pharmaceutical composition comprising about 0.5 to 200 mg, preferably about 1 to 50 mg of one or more of the benzothiazepine compounds per day for an average adult human depending on the activity of the particular compound chosen. The dosage may be divided into 1 to 4 unit dosage forms per day. While the therapeutic methods of the invention are most useful for human subjects in need of alleviation of hypertension or angina, the compounds may be administered to other mammals at comparable dosages per weight of the subject.

Pharmaceutical compositions containing the benzothiazepine compounds of the present invention of formula (I), an acid addition salt thereof or a quaternary ammonium compound thereof as the active ingredient may be prepared by intimately mixing the compound with a pharmaceutical carrier according to conventional pharmaceutical compounding techniques, which carrier may take a wide variety of forms depending on the form of preparation desired for administration, e.g. oral or parenteral. In preparing the compositions in oral dosage form, any of the usual pharmaceutical media may be employed, including liquid carriers such as water, glycols, oils, alcohols and the like for oral liquid preparations such as suspensions, elixirs and solutions; and solid carriers such as starches, sugars, kaolin, calcium stearate, ethyl cellulose, etc., including materials which function as lubricants, binders, disintegrating agents and the like for powders, capsules and tablets. Because of their ease in administration, tablets and capsules represent the most advantageous oral dosage form. These compositions employ solid pharmaceutical carriers such as the aforementioned starches, sugars, kaolin and the like, generally with a lubricant such as calcium stearate.

It is especially advantageous to formulate the aforementioned pharmaceutical composition in dosage unit form for ease of administration and uniformity of dosage. The term "dosage unit form" as used in the specification and claims herein refers to physically discrete units suitable as unitary dosages, each unit containing a predetermined quantity of active ingredient calculated to produce the desire therapeutic effect in association with the required pharmaceutical carrier. Examples of such dosage unit forms are tablets, capsules, pills, powder packets, wafers, teaspoonful, tablespoonful and the like, and segregated multiples thereof.

Also part of the present invention are novel intermediates.

In the following Examples, the following abbreviations are used: E (trans); Z (cis); bp (boiling point); mp (melting point); g (grams); ml (milliliters); glc (gas liquid chromatography); hplc (high pressure liquid chromatography); M (molar); µM (micromolar); THF (tetrahydrofuran); MeOH (methanol); i-PrOH (isopropanol); DMF (dimethylformamide); EtOAc (ethyl acetate); DMSO (dimethylsulfoxide); RT (room temperature); mmoles (millimoles); mg (milligrams); mm (millimeters); hr (hours); min (minutes); and C, H, N etc. (the chemical symbols for the elements). Unless otherwise indicated, all temperatures are reported in degrees centigrade (° C), all pressures in mm of mercury and all references to ether are to diethyl ether.

## Example 1

a. 2-(3-Methoxyphenyl)ethyl methanesulfonate

To a flask under argon was added 10.1 g (0.0661 mole) 3-methoxyphenethyl alcohol, 150 mL methylene chloride and 9.2 ml (0.0661 mole) triethylamine. The solution was cooled to 0° C and 5.1 ml (0.0661 mole) methanesulfonyl chloride was added slowly. The reaction mixture was warmed to room temperature. After 24 hr, the reaction was washed with water and brine, and dried with $MgSO_4$. The solvent was evaporated in vacuo to give 15.0 g of the product as a clear oil.

b. 3-Methoxyphenethyl iodide

To a flask under argon was added 14.6 g (0.0634 mole) 2-(3-methoxyphenyl)ethyl methanesulfonate in 400 ml of acetone and 36.5 g (0.243 mole) of sodium iodide. The reaction mixture was refluxed for 2.5 hr. The acetone was evaporated in vacuo and the residue dissolved in ether. The ether was washed with water, sodium bicarbonate, sodium thiosulfate, water and brine, dried over $MgSO_4$, and evaporated in vacuo to give a light green oil. Distillation in a Kugelrohr apparatus gave 14.6 g of the desired product as a clear oil.

c. cis-3-(Acetyloxy)-2,3,4,5-tetrahydro-2-(4-methoxyphenyl)-N-[2-(3-methoxyphenyl)ethyl]-N,N-dimethyl-4-oxo-1,5-benzothiazepine-5-ethanaminium iodide

To a flask under argon was added 5.0 g (0.0121 mole) cis-3-(acetyloxy)-5-[2,(N,N-dimethylamino)ethyl]-2,3-dihydro-2-(4-methoxyphenyl)-1,5-benzothiazepine-4(5H)-one (diltiazem), 25 ml EtOAc, and 3.8 g (0.0145 mole) 3-methoxyphenethyl iodide. The reaction mixture was heated between 60-65° C for 96 hr then filtered. One recrystallization from 95% ethanol gave a 4.44 g mixture of the product and diltiazem hydroiodide as a white solid.

d. cis-3-(Acetyloxy)-5-[2-[[2-(3-methoxyphenyl)ethyl]methylamino]ethyl]-2,3-dihydro-2-(4-methoxyphenyl)-1,5-benzothiazepine-4(5H)-one (E)-Butenedioate (1:1)

To a flask under argon was added 0.77 g (0.0193 mole) of 60% NaH in oil. The NaH was washed with hexane and 50 ml of DMF was added. To the suspension was slowly added 2.0 ml (0.0193 mole) of thiophenol. The solution was stirred 5 min and 4.36 g (0.0064 mole) of crude cis-3-(acetyloxy)-2,3,4,5-tetrahydro-2-(4-methoxyphenyl)-N-[2-(3-methoxyphenyl)ethyl]-N,N-dimethyl-4-oxo-1,5-benzothiazepine-5-ethanaminium iodide added. After heating at 80° C for 4 hr, the DMF was evaporated. The residue was dissolved in ether and washed with water, sodium bicarbonate, water and brine, dried over $K_2CO_3$ and evaporated. The residue was purified by flash chromatography, eluting with 1:50 MeOH:$CHCl_3$, to give the pure title product as a free base. Combining the product with one equivalent of fumaric acid in 2-propanol gave 2.72 g of the title compound, mp 136-141° C.

## Example 2

cis-3-Hydroxy-5-[2-[[2-(3-methoxyphenyl)ethyl]methylamino]ethyl]2,3-dihydro-2-(4-methoxyphenyl)-1,5-benzothiazepine-4(5H)-one (E)-Butenedioate (1:1)

To a flask under argon containing 4.31 g (8.07 mmole) cis-3-(acetyloxy)-5-[2-[[3-methoxyphenyl)ethyl]-methylamino]ethyl]-2,3-dihydro-2-(4-methoxyphenyl)1,5-benzothiazepine-4(5H)-one was added 35 ml methanol, the product of Example 1d, and 0.59 g (8.88 mmole) 85% KOH. After stirring at room temperature for 72 hr, the methanol was evaporated in vacuo. The residue was dissolved in ether and washed with water and brine, dried over $K_2CO_3$ and evaporated in vacuo. The residue was purified by flash chromatography, eluting with 1% methanol in chloroform. The purified residue was combined with one equivalent of fumaric acid in 2-propanol to give 3.05 g of the title product, mp 169-171 °C.

Example 3

a. 2-(3,4-Dimethoxyphenyl)ethyl methanesulfonate

A solution of 18.2 g (0.1 mole) of 3,4-dimethoxyphenethyl alcohol in 150 ml of methylene chloride was cooled to -5 °C. To the solution was added 13.9 ml (0.1 mole) of triethylamine followed by 7.7 ml (0.1 mole) of methanesulfonyl chloride keeping the temperature below 0 °C. The reaction was stirred for 4 hr, poured into water, washed with brine, dried over $Na_2SO_4$, and evaporated to give 26.7 g of product as a yellow oil.

b. 3,4-Dimethoxyphenethyl iodide

To a flask was added 21.2 g (0.078 mole) of 2-(3,4-dimethoxyphenyl)ethyl methanesulfonate and 44.65 g (0.3 mole) of sodium iodide in 420 ml of acetone. The solution was refluxed for 2 hr, poured into water, washed with brine, dried over $MgSO_4$, and evaporated to give 21.1 g of a yellow oil. The oil was recrystallized in MeOH to give 17.1 g of product, mp 43-46 °C.

c. cis-3-(Acetyloxy)-2,3,4,5-tetrahydro-2-(4-methoxyphenyl)-N-[2-(3,4-dimethoxyphenyl)ethyl]N,N-dimethyl-4-oxo-1,5-benzothiazepine-5-ethanaminium iodide

To a flask under argon containing 4.94 g (0.0119 mole) diltiazem was added 50 ml ethyl acetate and 3.48 g (0.0119 mole) 3,4-dimethoxyphenethyl iodide. The reaction was refluxed for 24 hr then filtered. The filtrate was washed with sodium bicarbonate and brine, dried over $K_2CO_3$, and 0.85g 3,4-dimethoxyphenethyl iodide added and the reaction refluxed for 18 hr then filtered. The last step was repeated but 1.75 g of 3,4-dimethoxyphenethyl iodide was added and the reaction refluxed 5 hr. The filtered materials were combined and recrystallized from 95% ethanol to give a mixture of product and diltiazem hydroiodide.

d. cis-3-(Acetyloxy)-5-[2-[[2-(3,4-dimethoxyphenyl)ethyl]-methylamino]ethyl]-2,3-dihydro-2-(4-methoxyphenyl)-1,5-benzothiazepine-4(5H)-one Hydrochloride Hydrate (2:2:1)

Using the method of Example 1d, but substituting cis-3-(acetyloxy)-2,3,4,5-tetrahydro-2-(4-methoxyphenyl)-N-[2-(3,4-dimethoxyphenyl)ethyl]-N,N-dimethyl-4-oxo-1,5-benzothiazepine-5-ethanaminium methanesulfonate for cis-3-(acetyloxy)-2,3,4,5-tetrahydro-2-(4-methoxyphenyl)-N-[2-(3-methoxyphenyl)ethyl]-N,N-dimethyl-4-oxo-1,5-benzothiazepine-5-ethanaminium iodide and using preparative hplc (1:3 acetone:hexane) for purification, product was obtained in 18% yield by precipitating from ethereal hydrogen chloride, mp 117-118 °C.

Example 4

cis-3-(Hydroxy)-5-[2-[[2-(3,4-dimethoxyphenyl)ethyl]methylamino]ethyl]-2,3-dihydro-2-(4-methoxyphenyl)-1,5-benzothiazepine-4(5H)-one Hydrochloride Hydrate (2:2:1)

Using the method of Example 2, but substituting cis-3-(acetyloxy)-5-[2-[[2-(3,4-dimethoxyphenyl)ethyl]-methylamino]ethyl]-2,3-dihydro-2-(4-methoxyphenyl)-1,5-benzothiazepine-4(5H)-one for cis -3-(acetyloxy)5-[2-[[2-(3-methoxyphenyl)ethyl]methylamino]ethyl]-2,3-dihydro-2-(4-methoxyphenyl)-1,5-benzothiazepine-4-(5H)-one, the title product was obtained in 57% yield by precipitation from ethereal hydrogen chloride after purification by preparation hplc (1:3 acetone:hexane), mp 110-115 °C.

Example 5

a. 2-(3,4-Dichlorophenyl)ethyl methansulfonate

Using the method of Example 1a, but substituting 3,4-dichlorophenethyl alcohol for 3-methoxyphenethyl alcohol, the title product was obtained as a yellow oil in 88% yield.

b. 3,4-dichlorophenethyl iodide

Using the method of Example 1b, but substituting 2-(3,4-dichlorophenyl)ethyl methanesulfonate for 2-(3-methoxyphenyl)ethyl methanesulfonate and refluxing the reaction for 24 hr, the title product was obtained as a yellow oil in 96% yield.

c. cis-3-(Acetyloxy)-2,3,4,5-tetrahydro-2-(4-methoxyphenyl)-N-[2-(3,4-dichlorophenyl)ethyl]-N,N-dimethyl-4-oxo-1,5-benzothiazepine-5-ethanaminium iodide

Using the method of Example 1c, but substituting 3,4-dichlorophenethyl iodide for 3-methoxyphenethyl iodide, heating to 58°C for 72 hr, and evaporating the ethyl acetate, the product mixture was crystallized from ether as a yellow solid.

d. cis-3-(Acetyloxy)-5-[2-[[2-(3,4-dichlorophenyl)ethyl]methylamino]ethyl]-2,3-dihydro-2-(4-methoxyphenyl)-1,5-benzothiazepine-4(5H)-one (E)-Butenedioate (1:1)

Using the method of Example 1d, but substituting cis-3-(acetyloxy)-2,3,4,5-tetrahydro-2-(4-methoxyphenyl)-N-]2-(3,4-dichlorophenyl)ethyl]-N,N-dimethyl-4-oxo-1,5-benzothiazepine-5-ethanaminium iodide for cis-3-(acetyloxy)-2,3,4,5-tetrahydro-2-(4-methoxyphenyl)-N-[2-(3-methoxyphenyl)ethyl]-N,N-dimethyl-4-oxo-1,5-benzothiazepine-5-ethanaminium iodide, the title product was obtained in 22% yield, mp 152-154°C.

Example 6

cis-3-(Hydroxy)-5-[2-[[3,4-dichlorophenyl)ethyl]methylamino]ethyl]-2,3-dihydro-2-(4-methoxyphenyl)-1,5-benzothiazepine-4(5H)-one (E)-Butenedioate (1:1)

Using the method of Example 2, but substituting cis-3-(acetyloxy)-5-[2-[[2-(3,4-dichlorophenyl)ethyl]-methylamino]ethyl]-2,3-dihydro-2-(4-methoxyphenyl)-1,5-benzothiazepine-4(5H)-one for cis-3-(acetyloxy)-5-[2-[[2-(3-methoxyphenyl)ethyl]methylamino]ethyl]-2,3-dihydro-2-(4-methoxyphenyl)-1,5-benzothiazepine-4-(5H)-one, the title product was obtained in 75% yield, mp 126-129°C.

Example 7

a. 2-(3,4-methylenedioxyphenyl)ethyl methanesulfonate

Using the method of Example 1a, but substituting 3,4-methylenedioxyphenethyl alcohol for 3-methoxyphenethyl alcohol and stirring for 4 hr at 0°C, the title product was obtained as a clear oil in 95% yield.

b. 3,4-Methylenedioxyphenethyl iodide

Using the method of Example 1b, but substituting 2-(3,4-methylenedioxyphenyl)ethyl methanesulfonate for 2-(3-methoxyphenyl)ethyl methanesulfonate, refluxing the reaction for only 2 hr and recrystallizing in 95% ethanol instead of distilling, the product was obtained in 64% yield, mp 36-38°C.

c. cis-3-(Acetyloxy)-2,3,4,5-tetrahydro-2-(4-methoxyphenyl)-N-[2-(3,4-methylenedioxyphenyl)ethyl]-N,N-dimethyl-4-oxo-1,5-benzothiazepine-5-ethanaminium iodide

Using the method of Example 1c, but substituting 3,4-methylenedioxyphenethyl iodide for 3-methoxyphenethyl iodide, stirring at room temperature for 72 hr followed by heating at 50°C for 48 hr, and decanting the solvent, the product mixture was obtained as an oil.

d. cis-3-(Acetyloxy)-5-[2-[[2-(3,4-methylenedioxyphenyl)ethyl]methylamino]ethyl]-2,3-dihydro-2-(4-methoxyphenyl)-1,5-benzothiazepine-4(5H)-one (E)-Butenedioate (1:1)

Using the method of Example 1d, but substituting cis-3-(acetyloxy)-2,3,4,5-tetrahydro-2-(4-methoxyphenyl)-N-[2,(3,4-methylenedioxyphenyl)ethyl]-N,N-dimethyl-4-oxo-1,5-benzothiazepine-5-ethanaminium iodide for cis-3-(acetyloxy)-2,3,4,5-tetrahydro-2-(4-methoxyphenyl)-N-[2-(3-methoxyphenyl)ethyl]-N,N-dimethyl-4-oxo-1,5-benzothiazepine-5-ethanaminium iodide, product was obtained in 30% yield, mp 158-160°C.

Example 8

cis-3-Hydroxy-5-[2-[[2-(3,4-methylenedioxyphenyl)ethyl]methylamino]ethyl]-2,3-dihydro-2-(4-methoxyphenyl)-1,5-benzothiazepine-4(5H)-one

In a manner similar to the procedure of Example 2, the title product may be obtained utilizing the 3-Acetyloxy starting material of Example 7d.

Example 9

a. 2-(3-Methylphenyl)ethyl methansulfonate

Using the method of Example 1a, but substituting 3-methylphenethyl alcohol for 3-methoxyphenethyl alcohol, the title product was obtained in 95% yield.

b. 3-Methylphenethyl iodide

Using the method of Example 1b. but substituting 2-(3-methylphenyl)ethyl methanesulfonate for 2-(3-methoxyphenyl)ethyl methanesulfonate and refluxing for 2 hr, the title product was obtained as a clear oil in 74% yield.

c. cis-3-(Acetyloxy)-2,3,4,5-tetrahydro-2-(4-methoxyphenyl)-N-[2-(3-methylphenyl)ethyl]-N,N-dimethyl-4-oxo-1,5-benzothiazepine-5-ethanaminium iodide

Using the method of Example 1c, but substituting 3-methylphenethyl iodide for 3-methoxyphenethyl iodide, heating at 65°C for 72 hr, and decanting the solvent, the product mixture was obtained as an oil.

d. cis-3-(Acetyloxy)-5-[2-[[2-(3-methylphenyl)ethyl]methylamino]ethyl]-2,3-dihydro-2-(4-methoxyphenyl)-1,5-benzothiazepine-4(5H)-one (E)-Butenedioate (1:1)

Using the method of Example 1d, but substituting cis-3-(acetyloxy)-2,3,4,5-tetrahydro-2-(4-methoxyphenyl)-N-[2-(3-methylphenyl)ethyl]-N,N-dimethyl-4-oxo-1,5-benzothiazepine-5-ethanaminium iodide for cis-3-(acetyloxy)-2,3,4,5-tetrahydro-2-(4-methoxyphenyl)-N-[2-(3-methoxyphenyl)ethyl]-N,N-dimethyl-4-oxo-1,5-benzothiazepine-5-ethanaminium iodide, the title product was obtained in 33% yield, mp 152-154°C.

Example 10

a. 2-(2-Methoxyphenyl)ethyl methanesulfonate

Using the method of Example 1a, but substituting 2-methoxyphenethyl alcohol for 3-methoxyphenethyl alcohol, the title product was obtained in 96% yield.

b. 2-Methoxyphenethyl iodide

Using the method of Example 1b, but substituting 2-(2-methoxyphenyl)ethyl methanesulfonate for 2-(3-methoxyphenyl)ethyl methansulfonate and refluxing the reaction for 24 hr, the title product was obtained in 88% yield.

c. cis-3-(Acetyloxy)-2,3,4,5-tetrahydro-2-(4-methoxyphenyl)-N-[2-(2-methoxyphenyl)ethyl]-N,N-dimethyl-4-oxo-1,5-benzothiazepine-5-ethanaminium iodide

The method of Example 1c was used substituting 2-methoxyphenethyl iodide for 3-methoxyphenethyl iodide. The reaction was heated at 65° C for 72 hr then filtered to give the title product.

d. cis-3-(Acetyloxy)-5-[2-[[2-methoxyphenyl)ethyl]methylamino]ethyl]-2,3-dihydro-2-(4-methoxyphenyl)1,5-benzothiazepine-4(5H)-one (E)-Butenedioate (1:1)

Using the method of Example 1d, but substituting cis-3-(acetyloxy)-2,3,4,5-tetrahydro-2-(4-methoxyphenyl)-N-[2-(2-methoxyphenyl)ethyl]-N,N-dimethyl-4-oxo-1,5-benzothiazepine-5-ethanaminium iodide for cis-3-(acetyloxy)-2,3,4,5-tetrahydro-2-(4-methoxyphenyl)-N-[2-(3-methoxyphenyl)ethyl]-N,N-dimethyl-4-oxo-1,5-benzothiazepine-5-ethanaminium iodide, the title product was obtained in 40% yield, mp 159-161° C.

Example 11

a. 2-(3,5-Dimethoxyphenyl)ethyl methansulfonate

Using the method of Example 1a, but substituting 3,5-dimethoxyphenethyl alcohol for 3-methoxyphenethyl alcohol and reacting to 0° C for 6 hr, the title product was obtained as a yellow oil in 100% yield.

b. 3,5-Dimethoxyphenethyl iodide

Using the method of Example 1b, but substituting 2-(3,5-dimethoxyphenyl)ethyl methansulfonate for 2-(3-methoxyphenyl)ethyl methanesulfonate and refluxing for 2 hr, the title product was obtained in 77% yield as a yellow solid, mp 30-33° C.

c. cis-3-(Acetyloxy)-2,3,4,5-tetrahydro-2-(4-methoxyphenyl)-N-[2-(3,5-dimethoxyphenyl)ethyl]-N,N-dimethyl-4-oxo-1,5-benzothiazepine-5-ethanaminium iodide

To a flask under nitrogen was added 5.0 g (0.0121 mole) of diltiazem, 3.52 g (0.0121 mole) of 3,5-dimethoxyphenethyl iodide, and 20 ml of EtOAc. The reaction mixture was heated at 50° C for 24 hr then at 75° C for 96 hr. The reaction was cooled to RT, the solvent decanted, and the oily residue washed three times with ETOAc. The oily residue was dissolved in methylene chloride, washed with water and dried over $MgSO_4$. The solvent was evaporated to give 5.45 g of a mixture of the title product and diltiazem hydroiodide as a tan foam.

d. cis-3-(Acetyloxy)-5-[2-[[2-(3,5-dimethoxyphenyl)ethyl]methylamino]ethyl]-2,3-dihydro-2-(4-methoxyphenyl)-1,5-benzothiazepine-4(5H)-one (E)-Butenedioate (1:1)

Using the method of Example 1d, but substituting cis-3-(acetyloxy)-2,3,4,5-tetrahydro-2-(4-methoxyphenyl)-N-[2-(3,5-dimethoxyphenyl)ethyl]-N,N-dimethyl-4-oxo-1,5-benzothiazepine-5-ethanaminium iodide for cis-3-(acetyloxy)-2,3,4,5-tetrahydro-2-(4-methoxyphenyl)-N[2-(3-methoxyphenyl)ethyl]-N,N-dimethyl-4-oxo-1,5-benzothiazepine-5-ethanaminium iodide and heating for 6 hr, product was obtained in 13% yield after recrystallizing from MeOH/i-PrOH, mp 179-181° C.

Example 12

a. 2-(2,3-Dichlorophenyl)ethyl methansulfonate

Using the method of Example 1a, but substituting 2,3-dichlorphenethyl alcohol for 3-methoxyphenethyl alcohol and reacting at 0° C for 4 hr, the title product was obtained as a yellow oil in 100% yield.

b. 2,3-Dichlorphenethyl iodide

Using the method of Example 1b, but substituting 2-(2,3-dichlorophenyl)ethyl methanesulfonate for 2-(3-methoxyphenyl)ethyl methanesulfonate and refluxing for 3 hr, the title product was obtained as a yellow oil in 67% yield.

c. cis-3-(Acetyloxy)-2,3,4,5-tetrahydro-2-(4-methoxyphenyl)-N-[2-(2,3-dichlorophenyl)ethyl]-N,N-dimethyl-4-oxo-1,5-benzothiazepine-5-ethanaminium iodide

Using the method of Example 1c, but substituting 2,3-dichlorophenethyl iodide for 3-methoxyphenethyl iodide and heating at 70° C for 96 hr, the title product was obtained as an off-white solid.

d. cis-3-(Acetyloxy)-5-[2-[[2,3-dichlorophenyl)ethyl]methylamino]ethyl]-2,3-dihydro-2-(4-methoxyphenyl)-1,5-benzothiazepine-4(5H)-one (E)-Butenedioate (1:1)

Using the method of Example 1d, but substituting cis-3-(acetyloxy)2,3,4,5-tetrahydro-2-(4-methoxyphenyl)-N-[2-(2,3-dichlorophenyl)ethyl]-N,N-dimethyl-4-oxo-1,5-benzothiazepine-5-ethanaminium iodide for cis-3-(acetyloxy)-2,3,4,5-tetrahydro-2-(4-methoxyphenyl)-N-[2-(3-methoxyphenyl)ethyl]-N,N-dimethyl-4-oxo-1,5-benzothiazepine-5-ethanaminium iodide, product was obtained in 14% yield, mp 145-147° C.

Example 13

a. (S)-N-(2-Hydroxy-1-methylethyl)-3,4-dimethoxybenzeneacetamide

Into a Morton flask was placed 10 g (0.137 mole) of (S)-(=)-2-amino-1-propanol in 200 mL of chloroform. To this was added 137 ml (0.137 mole) of 1N sodium hydroxide. To this mixture was added over 1 hr a solution of 29.4 g (0.137 mole) of 3,4-dimethoxyphenylacetyl chloride in 100 ml of chloroform. The reaction mixture was stirred vigorously for 4 hr. The organics were separated, washed with water, brine and dried ($K_2CO_3$). The solvent was evaporated in vacuo and the residue was recrystallized from ETOAc to give 22.14 g of (S)-N-(2-hydroxy-1-methylethyl)-3,4-dimethoxybenzeneacetamide (64% yield), mp 122-125° C.

b. (S)-2-[[2-(3,4-Dimethoxyphenyl)ethyl]amino]-1-propanol

To a suspension of 12.6 g (0.33 mole) lithium aluminum hydride (LAH) in 150 mL of dry THF under argon was added dropwise a suspension of 21.0 g (0.083 mole) (S)-N-(2-hydroxy-1-methylethyl)-3,4-dimethoxybenzeneacetamide in dry THF. The reaction mixture was heated under reflux for 4 hr. An additional 1.0 g of LAH was added and refluxed another 3 hr. An additional 3.0 g of LAH was added and the reaction was refluxed overnight. To the reaction was added 69 mL (0.48 mole) of triethanolamine over 1 hr. After stirring for 90 min, 16 ml of water was added and this was stirred overnight. An additional 12 ml of water was added. The solid that formed was filtered off and the filter cake was washed three times with ether and once with THF. The filtrate was washed with brine and dried ($K_2CO_3$). The solvent was removed in vacuo. The residue was dissolved in ether/acetonitrile, the undissolved solid was filtered off and the filtrate was evaporated in vacuo. The residue was treated with ethereal hydrogen chloride and the solid was collected. Three recrystallizations from acetonitrile gave 6.9 g of (S)-2-[[2-(3,4-dimethoxyphenyl)ethyl]-amino]-1-propanol (38%) mp. 120-123° C.

c. (S)-2-[[2-(3,4-Dimethoxyphenyl)ethyl]methylamino]-1-propanol

To a solution of 6.25 g of (0.026 mole) (S)-2-[[2-(3,4-dimethoxyphenyl)ethyl]amino]-1-propanol in 50 ml of MeOH under argon was added 3.9 ml (0.052 mole) of 37% aqueous formaldehyde and 4.7 g of sodiumborohydride pellets added one at a time. The solution was stirred at RT for 24 hr. an additional 1.0 ml of 37% aqueous formaldehyde and 1.0 g of sodium borohydride were added and the solution stirred for an additional 2.5 hr. Enough glacial acetic acid was added to react with the sodium borohydride. The reaction was made basic by addition of 3N sodium hydroxide. The methanol was evaporated in vacuo and the residue was extracted three times with ether. The organics were washed with brine an dried ($K_2CO_3$). Evaporation of the solvent in vacuo gave 5.74 g of (S)-2-[[2-(3,4-dimethoxyphenyl)ethyl]methylamino]-1-propanol as an oil (87% yield).

d. (S)-N-(2-Chloro-1-methylethyl)-3,4-dimethoxy-N-methylbenzeneethanamine Hydrochloride

To a solution of 7.06 g (0.028 mole) of (S)-2-[[2-(3,4-dimethoxyphenyl)ethyl]methylamino]-1-propanol hydrochloride in 100 ml of chloroform was added 4.8 ml (0.067 mole) of thionyl chloride and the reaction was heated to reflux for 3 hr. The reaction was cooled and the solvent was evaporated in vacuo to give 3.09

g of (S)-N-(2-chloro-1-methylethyl)-3,4-dimethoxy-N-methylbenzeneethaneamine hydrochloride as a brown glass (43%).

e. cis-3-(Acetyloxy)-5-[(S)-2-[[2-(3,4-dimethoxyphenyl)ethyl]methylamino]propyl]-2,3-dihydro-2-(4-methoxyphenyl)-1,5-benzothiazepin-4(5H)-one

Dry silica gel 1.32 g, 2.75 g (0.008 mole) of 2-(4'-methoxyphenyl)-3-acetoxy-2,3-dihydro-1,5-benzothiazepine-4(5H)-one, 28 mL of Aldrich gold label grade DMSO, and 0.39 g (0.0096 mole) of 60% sodium hydride in oil, was stirred for 1 hr, and 3.21 g (0.0096 mole) of (S)-N-(2-chloro-1-methylethyl)-3,4-dimethoxy-N-methylbenzeneethaneamine in dry ether was added. The reaction was stirred at RT for 5 hr. Glacial acetic acid was added until pH 6 and the reaction was stirred for 10 min. The reaction was extracted three times with $CH_2Cl_2$, the organics were combined, washed with water, brine and dried ($Na_2SO_4$). The solvent was evaporated in vacuo. The residue was flashed chromatographed on silica gel using 1:1 EtOAc:hexane as the elutant. The isolated material was treated with ethereal hydrogen chloride to give 2.24 g of the title product as the hydrochloride (50% yield), mp 147-150 °C.

Example 14

cis-3-(Acetyloxy)-5-[(S)-2-[[2-(3,4-dimethoxyphenyl)ethyl]methylamino]-1-methylethyl]-2,3-dihydro-2-(4-methoxyphenyl)-1,5-benzothiazepin-4(5H)-one Hydrochloride

From the chromatography in Example 13c, a separate fraction was also isolated. This was treated with ethereal hydrogen chloride to give 0.14 g of the title compound (3% yield), mp 125-128 °C.

Example 15

a. 1,2-Dichloro-3-(2-methoxyethenyl)benzene

Into a flask under argon containing 700 ml of dry THF was placed 159 g (0.47 mole) of methyl (triphenylphosphoranylidene)acetate. To the stirred suspension cooled in an ice/water bath was added dropwise 320 ml (0.47 mole) of 1.45 M n-butyllithium in hexane. After stirring for 20 min, 74 g (0.42 mole) of 2,3-dichlorobenzaldehyde in 700 ml of dry THF was added. After 2 hr the reaction mixture was washed with an ammonium chloride solution, brine and dried ($MgSO_4$). The solvent was evaporated in vacuo. The residue was taken up in ether, the undissolved solid was filtered off, and the solvent was evaporated in vacuo. The residue was distilled in a Kugelrohr apparatus at 0.005 mm Hg (0.67 Pa) and the fraction distilling between 80-100 °C collected. The distillate was distilled at 0.005 mm Hg(0.67 Pa) using a Vigro column and the product collected between 101-116 °C. A preparative hplc on silica gel using hexane as the elutant gave 28.02 g of the title compound as an oil (33%).

b. 2,3-Dichlorobenzeneacetaldehyde

To a solution of 15.0 g (0.074 mole) 1,2-dichloro-3-(2-methoxyethenyl)benzene in 150 ml of THF was added 15 ml of 35% perchloric acid in water. The reaction was refluxed for 3.5 hr then poured into ice/water. The aqueous layer was extracted two times with ether, the organics were washed with water, brine and dried ($MgSO_4$). Evaporation of the solvent gave 13.91 g of crude 2,3-dichlorobenzeneacetaldehyde a yellow oil (99%).

c. (S)-2-[[2-(2,3-Dichlorophenyl)ethyl]amino]-1-propanol Hydrogenchloride

To a solution of 13.0 g (0.074 mole) of crude 2,3-dichlorobenzeneacetaldehyde in 50 ml of MeOH was added 5.7 ml (0.074 mole) of (S)-2-amino-1-propanol. The reaction was placed under an atmosphere of argon and 3.6 g (0.058 mole) of sodium cyanoborohydride was added. After stirring for 2.5 hr, the reaction was made acidic by adding ethereal hydrogen chloride. After stirring of 30 min enough 3N sodium hydroxide was added to make the reaction basic. The reaction mixture was extracted with ether and the ether layer was washed with water, brine and dried ($K_2CO_3$). Evaporation of the solvent in vacuo gave a yellow oil. The oil was treated with ethereal hydrogen chloride and the solid was collected. The solid was recrystallized from 2-propanol to give 5.10 g of the title compound (28% yield), mp 148-151 °C.

d. (S)-2-[[2-(2,3-dichlorophenyl)ethyl]methylamino]-1-propanol Hydrochloride

According to the procedure in Example 13c, the title compound was prepared (77% yield) mp 94-97° C.

e. (S)-2,3-dichloro-N-(2-chloro-1-methylethyl)-N-methylbenzeneethaneamine Hydrochloride

According to the method in Example 13d, the title compound was prepared (96.5% yield), mp 157-158° C.

f. cis-3-(Acetyloxy)-5-[(S)-2-[[2-(2,3-dichlorophenyl)ethyl]methylamino]propyl]-2,3-dihydro-2-(4-methoxyphenyl)-1,5-benzothiazepin-4(5H)-one

By the procedure of Example 3c and employing (S)-2,3-dichloro-N-(2-chloro-1-methyethyl)-N-methylbenzeneethaneamine in place of (S)-N-(2-chloro-1-methylethyl)-3,4-dimethoxy-N-methylbenzeneethaneamine, the title compound was obtained, m/e = 587 by mass spectrometry.

Example 16

cis-3-(Acetyloxy)-5-[2-[[2-(3,4-dimethoxyphenyl)ethyl]methylamino]ethyl]-2,3-dihydro-2-(4-methoxyphenyl)-1,5-benzothiazepine-4(5H)-one Hydrochloride Hydrate

Formula (I): X = H; $R^1$ = 4-$OCH_3$phenyl; $R^2$ = -$COCH_3$; Y = -$CH_2CH_2$-; $R^3$-$CH_3$; p = 0; Alk = -$CH_2CH_2$-; q = 0; Ar = 3,4-di-$OCH_3$phenyl

To a solution of 6.5 g (0.0157 mole) cis-3-(acetyloxy)-5-[2-(N,N-dimethylamino)ethyl]-2,3-dihydro-2-(4-methoxyphenyl)-1,5-benzothiazepine-4(5H)-one, known as diltiazem, in 25 mL of dry DMF was added 3.8 g (0.0172 mole) 3,4-dimethoxyphenethylbromide. The solution was heated to 100° C for 10 hr then left to stir at room temperature for 60 hr. An additional 1.9 g (0.00775 mole) of the bromide was added. The reaction was heated for 35 hr at 100° C at which point the quaternary salt formation was complete.

To 1.05 g (0.0157 mole) of NaH in 10 mL of dry DMF was added 4.81 mL (0.0157 mole) thiophenol. After stirring for 20 min at room temperature the reaction mixture containing the quaternary salt was added and the resulting solution was heated to 80° C for 4 hr. The reaction mixture was cooled and the solvent was concentrated in vacuo. The residue was partitioned between ether and aqueous $NaHCO_3$. The aqueous layer was again extracted with ether and the ether layers were combined, washed with $H_2O$, $NaHCO_3$, brine and dried ($K_2CO_3$). The ether was evaporated in vacuo to give 11.03 g of a yellow oil. Flash chromatography with silica gel was performed twice on the oil first eluting with 1:1 acetone:hexane then 1:3 acetone:hexane. Fractions containing the desired product were concentrated in vacuo. To the resulting oil was added ethereal HCl to give 0.600 g of cis-3-(acetyloxy)-5-[2[[2-(3,4-dimethoxyphenyl)ethyl]-methylamino]ethyl]-2,3-dihydro-2-(4-methoxyphenyl)-1,5-benzothiazepine-4(5H)-one hydrochloride hydrate; mp 118-122° C; yield 6.8%.

Examples 17-22

Using the procedures of Example 16 and substituting the appropriate compounds of formula (III) in the place of 3,4-dimethoxyphenethylbromide, the following compounds may be obtained:
cis-3-(acetyloxy)-5-[2-[[2-(3,5-dimethoxyphenyl)ethyl]methylamino]ethyl]-2,3-dihydro-2-(4-methoxyphenyl)-1,5-benzothiazepine-4(5H)-one;
cis-3-(acetyloxy)-5-[2-[[2-(3,4-dichlorophenyl)ethyl]methylamino]ethyl]-2,3-dihydro-2-(4-methoxyphenyl)-1,5-benzothiazepine-4(5H)-one;
cis-3-(acetyloxy)-5-[2-[[2-(2,3-dichlorophenyl)ethyl]methylamino]ethyl]-2,3-dihydro-2-(4-methoxyphenyl)-1,5-benzothiazepine-4(5H)-one;
cis-3-(acetyloxy)-5-[2-[[2-(3-methoxyphenyl)ethyl]methylamino]ethyl]-2,3-dihydro-2-(4-methoxyphenyl)-1,5-benzothiazepine-4(5H)-one;
cis-3-(acetyloxy)-5-[2-[[2-(3-trifluoromethylphenyl)ethyl]methylamino]ethyl]-2,3-dihydro-2-(4-methoxyphenyl)-1,5-benzothiazepine-4(5H)-one; and
cis-3-(acetyloxy)-5-[2-[[2-(3,4-dimethoxyphenyl)propyl]methylamino]ethyl]-2,3-dihydro-2-(4-methoxyphenyl)-1,5-benzothiazepine-4(5H)-one.

Example 23

cis-5-[2-[[2-(3,4-Dimethoxyphenyl)ethyl]methylamino]ethyl]-2,3-dihydro-3-hydroxy-2-(4-methoxyphenyl)-1,5-benzothiazepine-4(5H)-one

The product of Example 16 may be hydrolyzed with sodium hydroxide to produce the title compound. Alternatively, the benzothiazepine starting material in Example 16 may be 2-(4-methoxyphenyl)-3-hydroxy-5-(β-dimethylaminoethyl)-2,3-dihydro-1,5-benzothiazepine-4(5H)-one as described in U.S. Patent 3,562,257 whereby the title compound will be produced.

## Claims

### Claims for the following Contracting States: BE, CH, DE, FR, GB, IT, LI, LU, NL, SE

1. A benzothiazepine of the following formula (I):

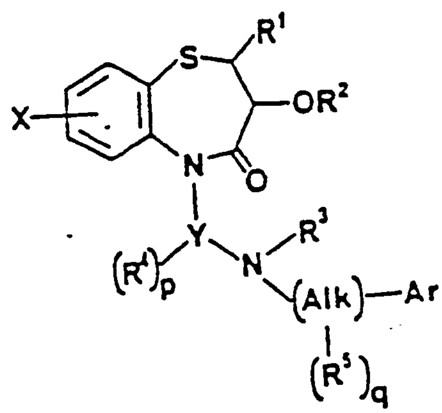

Wherein

| | |
|---|---|
| X | is a hydrogen atom or a halogen atom; |
| $R^1$ | is phenyl or phenyl independently substituted by 1 to 3 of methyl, ethyl, n-propyl, isopropyl, n-butyl, sec-butyl, tert-butyl, iso-butyl, methoxy, ethoxy, n-propoxy, iso-propoxy, n-butoxy, sec-butoxy, tert-butoxy, iso-butoxy, or halogen; |
| $R^2$ | is hydrogen, acetyl, propionyl, 2-methyl-propionyl or butyryl; |
| Y | is straight chain alkylene of 2 or 3 carbons; |
| $R^3$ | is methyl, ethyl, n-propyl, iso-propyl, n-butyl, sec-butyl or iso-butyl; |
| $R^4$ | is independently methyl, ethyl, n-propyl, iso-propyl, n-butyl, sec-butyl or iso-butyl; |
| p | is 0, 1, 2 or 3; |
| Alk | is ethylene, trimethylene or tetramethylene; |
| $R^5$ | is hydroxy, alkyl of 1 to 4 carbons or phenyl; |
| q | is 0, 1, 2 or 3; and |
| Ar | is phenyl, phenoxy, thiophenoxy, naphthyl or a 5- or 6-membered heterocyclic aromatic ring having one nitrogen, sulfur or oxygen atom which rings may be substituted independently by one or more of alkyl, alkoxy or alkylthio of 1 to 6 carbons each, hydroxy, halogen, fluoroalkyl of 1 to 6 carbons, nitro, amino, dialkylamino of 2 to 12 carbons or methylenedioxy at adjacent ring carbons, |

and the pharmaceutically acceptable acid addition salts and quaternary ammonium compounds thereof.

2. The benzothiazepine of claim 1 wherein X is hydrogen.

3. The benzothiazepine of claim 1 or 2 wherein Alk is ethylene.

4. The benzothiazepine of any one of claims 1 to 3, wherein

| | |
|---|---|
| X | is hydrogen; |
| $R^1$ | is phenyl substituted by $C_{1-4}$ alkoxy; |
| $R^2$ | is alkanoyloxy of up to 4 carbon atoms; |
| Y | is ethylene; |
| $R^3$ | is $C_{1-4}$ alkyl; |

p is 0;
Alk is ethylene;
q is 0; and
Ar is phenyl or phenyl substituted independently by 1 or 2 of $C_{1-6}$ alkyl, $C_{1-6}$ alkoxy, $C_{1-6}$ alkylthio, hydroxy, halogen, fluoro-$C_{1-6}$alkyl, nitro, amino or $C_{2-12}$ dialkylamino or by methylene dioxy at adjacent ring carbons.

**5.** The benzothiazepine of Claim 1, wherein said benzothiazepine is:

3-hydroxy-5-[2-[[2-(3-methoxyphenyl)ethyl]methylamino]ethyl]-2,3-dihydro-2-(4-methoxyphenyl)-1,5-benzothiazepine-4(5H)-one;
3-(hydroxy)-5-[2-[[3,4-dichlorophenyl)ethyl]methylamino]ethyl]-2,3-dihydro-2-(4-methoxyphenyl)-1,5-benzothiazepine-4(5H)-one;
3-(acetyloxy)-5-[2-[[2-(3,4-methylenedioxyphenyl)ethyl]methylamino]ethyl]-2,3-dihydro-2-(4-methoxyphenyl)-1,5-benzothiazepine-4(5H)-one;
3-hydroxy-5-[2-[[2-(3,4-methylenedioxyphenyl)ethyl]methylamino]ethyl]-2,3-dihydro-2-(4-methoxyphenyl)-1,5-benzothiazepine-4(5H)-one;
3-(acetyloxy)-5-[2-[[2-(3-methylphenyl)ethyl]methylamino]ethyl]-2,3-dihydro-2-(4-methoxyphenyl)-1,5-benzothiazepine-4(5H)-one;
3-(acetyloxy)-5-[2-[[2-methoxyphenyl)ethyl]methylamino]ethyl]-2,3-dihydro-2-(4-methoxyphenyl)1,5-benzothiazepine-4(5H)-one;
3-(acetyloxy)-5-[(S)-2-[[2-(3,4-dimethoxyphenyl)ethyl]methylamino]-1-methylethyl]-2,3-dihydro-2-(4-methoxyphenyl)-1,5-benzothiazepin-4(5H)one;
3-(acetoxy)-5-[(S)-2-[[2-(2,3-dichlorophenyl)ethyl]methylamino]propyl]-2,3-dihydro-2-(4-methoxyphenyl)-1,5-benzothiazepin-4(5H)-one;
3-(acetyloxy)-5-[2-[[2-(3,4-dimethoxyphenyl)ethyl]methylamino]ethyl]-2,3-dihydro-2-(4-methoxyphenyl)-1,5-benzothiazepine-4(5H)-one;
3-(acetyloxy)-5-[2-[[2-(3,5-dimethoxyphenyl)ethyl]methylamino]ethyl]-2,3-dihydro-2-(4-methoxyphenyl)-1,5-benzothiazepine-4(5H)-one;
3-(acetyloxy)-5-[2-[[2-(3,4-dichlorophenyl)ethyl]methylamino]ethyl]-2,3-dihydro-2-(4-methoxyphenyl)-1,5-benzothiazepine-4(5H)-one;
3-(acetyloxy)-5-[2-[[2-(2,3-dichlorophenyl)ethyl]methylamino]ethyl]-2,3-dihydro-2-(4-methoxyphenyl)-1,5-benzothiazepine-4(5H)-one;
3-(acetyloxy)-5-[2-[[2-(3-methoxyphenyl)ethyl]methylamino]ethyl]-2,3-dihydro-2-(4-methoxyphenyl)-1,5-benzothiazepine-4(5H)-one;
3-(acetyloxy)-5-[2-[[2-(3-dimethylaminophenyl)ethyl]methylamino]ethyl]-2,3-dihydro-2-(4-methoxyphenyl)-1,5-benzothiazepine-4(5H)-one;
3-(acetyloxy)-5-[2-[[2-(3-trifluoromethylphenyl)ethyl]methylamino]ethyl]-2,3-dihydro-2-(4-methoxyphenyl)-1,5-benzothiazepine-4(5H)-one;
3-(acetyloxy)-5-[2-[[2-(3,4-dimethoxyphenyl)propyl]methylamino]ethyl]-2,3-dihydro-2-(4-methoxyphenyl)-1,5-benzothiazepine-4(5H)-one;
5-[2-[[2-(3,4-dimethoxyphenyl)ethyl]methylamino]ethyl]-2,3-dihydro-3-hydroxy-2-(4-methoxyphenyl)-1,5-benzothiazepine-4(5H)-one; or
3-(acetyloxy)-5-[2-[[2-(3,4-dimethoxyphenyl)ethyl]methylamino]propyl]-2,3-dihydro-2-(4-methoxyphenyl)-1,5-benzothiazepine-4(5H)-one.

**6.** The compound of the following formula (VI):

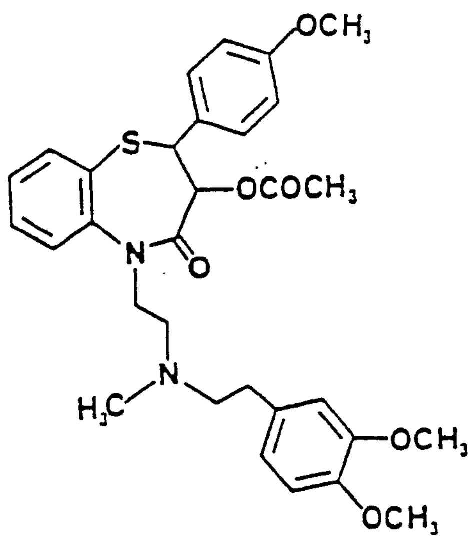

(VI)

or formula (VII)

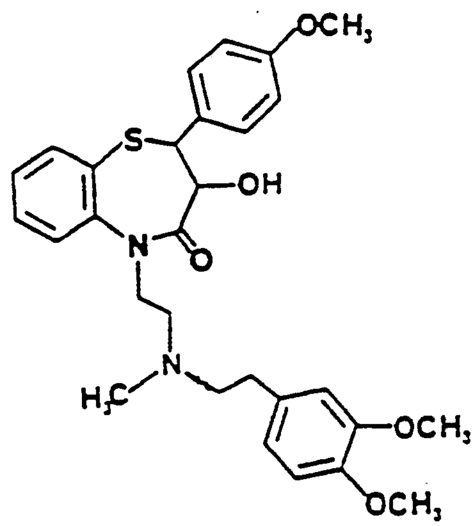

(VII)

or a pharmaceutically acceptable acid addition salt thereof.

7. The benzothiazepine of any one of claims 1 to 6 wherein said benzothiazepine is the cis isomer.

8. A pharmaceutical composition comprising a benzothiazepine of any one of claims 1 to 7 in combination with a pharmaceutically acceptable diluent or carrier.

9. The benzothiazepine of any one of claims 1 to 7 or the pharmaceutical composition of claim 8 for use in treating angina pectoris or hypertension, or for preventing the reoccurrence of heart attacks, in a mammal.

**Claims for the following Contracting States: AT, ES, GR**

1. A process for the preparation of a benzothiazepine of the following formula (I):

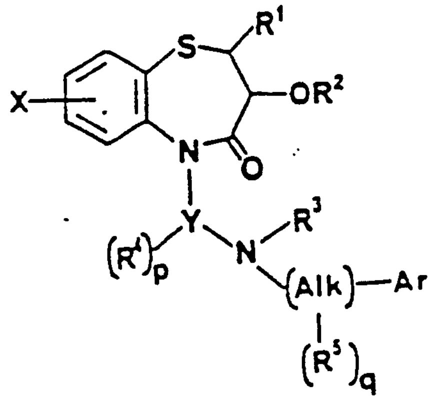

(I)

Wherein

- X is a hydrogen atom or a halogen atom;
- $R^1$ is phenyl or phenyl independently substituted by 1 to 3 of methyl, ethyl, n-propyl, isopropyl, n-butyl, sec-butyl, tert-butyl, iso-butyl, methoxy, ethoxy, n-propoxy, iso-propoxy, n-butoxy, sec-butoxy, tert-butoxy, iso-butoxy, or halogen;
- $R^2$ is hydrogen, acetyl, propionyl, 2-methyl-propionyl or butyryl;
- Y is straight chain alkylene of 2 or 3 carbons;
- $R^3$ is methyl, ethyl, n-propyl, iso-propyl, n-butyl, sec-butyl or iso-butyl;
- $R^4$ is independently methyl, ethyl, n-propyl, iso-propyl, n-butyl, sec-butyl or iso-butyl;
- p is 0, 1, 2 or 3;
- Alk is ethylene, trimethylene or tetramethylene;
- $R^5$ is hydroxy, alkyl of 1 to 4 carbons or phenyl;
- q is 0, 1, or 2 or 3; and
- Ar is phenyl, phenoxy, thiophenoxy, naphthyl or a 5- or 6-membered heterocyclic aromatic ring having one nitrogen, sulfur or oxygen atom which rings may be substituted independently by one or more of alkyl, alkoxy or alkylthio of 1 to 6 carbons each, hydroxy, halogen, fluoroalkyl of 1 to 6 carbons, nitro, amino, dialkylamino of 2 to 12 carbons or methylenedioxy at adjacent ring carbons,

and the pharmaceutically acceptable acid addition salts and quaternary ammonium compounds thereof, comprising:
reacting a compound of the following formula (A)

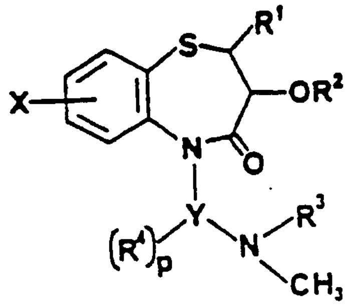

(A)

wherein
$X, Y, R^1, R^2, R^3, R^4$ and p are as defined above for formula (I) with a compound of the following formula (B)

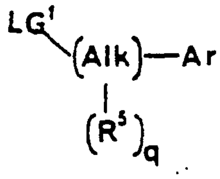

(B)

wherein
$R^5$, q, Alk and Ar are as defined above for formula (I); and $LG^1$ is a suitable leaving group, at about room temperature up to 150 ° C, to form a quarternary ammonium salt conjugate and then reacting the conjugate with a strong nucleophile and optionally converting the product to a pharmaceutically acceptable acid addition salt or a quaternary ammonium compound thereof.

2. A process according to claim 1 wherein the leaving group is tosyl, iodo, mesyl or bromo.

3. A process for the preparation of a benzothiazepine of the formula (I) as defined in claim 1, and the pharmaceutically acceptable acid addition salts and quaternary ammonium compounds thereof, comprising:
reacting a compound of the following formula (C)

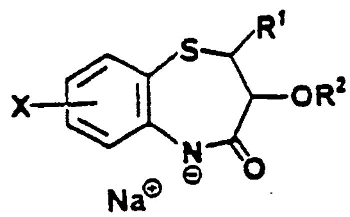

(C)

wherein
X,$R^1$ and $R^2$ are as defined in claim 1 for formula (I) with a compound of the following formula (D)

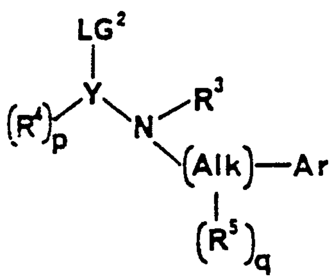

(D)

wherein
$R^3$,$R^4$,$R^5$,p,q, Alk, Ar and Y are as defined in claim 1 for formula (I); and $LG^2$ is a suitable leaving group, at a temperature of about 25 to 150 ° C, and optionally converting the product to a pharmaceutically acceptable acid addition salt or a quaternary ammonium compound thereof.

4. A process according to claim 3 wherein the leaving group is chlorine or bromine.

5. A process for the preparation of a pharmaceutical composition comprising mixing in a pharmaceutically effective amount a benzothiazepine of the formula (I) as defined in claim 1, or a pharmaceutically acceptable acid addition salt or quaternary ammonium compound thereof with a pharmaceutically acceptable diluent, carrier or excipient.

6. A process according to any one of claims 1 to 5 wherein X is hydrogen.

7. A process according to any one of claims 1 to 6 wherein Alk is ethylene.

8. A process according to any one of claims 1 to 7 wherein
X is hydrogen;
$R^1$ is phenyl substituted by $C_{1-4}$ alkoxy;
$R^2$ is alkanoyloxy of up to 4 carbon atoms;
Y is ethylene;
$R^3$ is $C_{1-4}$ alkyl;
p is 0;
Alk is ethylene;
q is 0; and
Ar is phenyl or phenyl substituted independently by 1 or 2 of $C_{1-6}$ alkyl, $C_{1-6}$ alkoxy, $C_{1-6}$ alkylthio, hydroxy, halogen, fluoro-$C_{1-6}$alkyl, nitro, amino or $C_{1-12}$ dialkylamino or by methylene dioxy at adjacent ring carbons.

9. A process according to any one of claims 1 to 5 claim wherein said benzothiazepine is:

3-hydroxy-5-[2-[[2-(3-methoxyphenyl)ethyl]methylamino]ethyl]-2,3-dihydro-2-(4-methoxyphenyl)-1,5-benzothiazepine-4(5H)-one;
3-(hydroxy)-5-[2-[[3,4-dichlorophenyl)ethyl]methylamino]ethyl]-2,3-dihydro-2-(4-methoxyphenyl)-1,5-benzothiazepine-4(5H)-one;
3-(acetyloxy)-5-[2-[[2-(3,4-methylenedioxyphenyl)ethyl]methylamino]ethyl]-2,3-dihydro-2-(4-methoxyphenyl)-1,5-benzothiazepine-4(5H)-one;
3-hydroxy-5-[2-[[2-(3,4-methylenedioxyphenyl)ethyl]methylamino]ethyl]-2,3-dihydro-2-(4-methoxyphenyl)-1,5-benzothiazepine-4(5H)-one;
3-(acetyloxy)-5-[2-[[2-(3-methylphenyl)ethyl]methylamino]ethyl]-2,3-dihydro-2-(4-methoxyphenyl)-1,5-benzothiazepine-4(5H)-one;
3-(acetyloxy)-5-[2-[[2-methoxyphenyl)ethyl]methylamino]ethyl]-2,3-dihydro-2-(4-methoxyphenyl)1,5-benzothiazepine-4(5H)-one;
3-(acetyloxy)-5-[(S)-2-[[2-(3,4-dimethoxyphenyl)ethyl]methylamino]-1-methylethyl]-2,3-dihydro-2-(4-methoxyphenyl)-1,5-benzothiazepin-4(5H)one;
3-(acetoxy)-5-[(S)-2-[[2-(2,3-dichlorophenyl)ethyl]methylamino]propyl]-2,3-dihydro-2-(4-methoxyphenyl)-1,5-benzothiazepin-4(5H)-one;
3-(acetyloxy)-5-[2-[[2-(3,4-dimethoxyphenyl)ethyl]methylamino]ethyl]-2,3-dihydro-2-(4-methoxyphenyl)-1,5-benzothiazepine-4(5H)-one;
3-(acetyloxy)-5-[2-[[2-(3,5-dimethoxyphenyl)ethyl]methylamino]ethyl]-2,3-dihydro-2-(4-methoxyphenyl)-1,5-benzothiazepine-4(5H)-one;
3-(acetyloxy)-5-[2-[[2-(3,4-dichlorophenyl)ethyl]methylamino]ethyl]-2,3-dihydro-2-(4-methoxyphenyl)-1,5-benzothiazepine-4(5H)-one;
3-(acetyloxy)-5-[2-[[2-(2,3-dichlorophenyl)ethyl]methylamino]ethyl]-2,3-dihydro-2-(4-methoxyphenyl)-1,5-benzothiazepine-4(5H)-one;
3-(acetyloxy)-5-[2-[[2-(3-methoxyphenyl)ethyl]methylamino]ethyl]-2,3-dihydro-2-(4-methoxyphenyl)-1,5-benzothiazepine-4(5H)-one;
3-(acetyloxy)-5-[2-[[2-(3-dimethylaminophenyl)ethyl]methylamino]ethyl]-2,3-dihydro-2-(4-methoxyphenyl)-1,5-benzothiazepine-4(5H)-one;
3-(acetyloxy)-5-[2-[[2-(3-trifluoromethylphenyl)ethyl]methylamino]ethyl]-2,3-dihydro-2-(4-methoxyphenyl)-1,5-benzothiazepine-4(5H)-one;
3-(acetyloxy)-5-[2-[[2-(3,4-dimethoxyphenyl)propyl]methylamino]ethyl]-2,3-dihydro-2-(4-methoxyphenyl)-1,5-benzothiazepine-4(5H)-one;
5-[2-[[2-(3,4-dimethoxyphenyl)ethyl]methylamino]ethyl]-2,3-dihydro-3-hydroxy-2-(4-methoxyphenyl)-1,5-benzothiazepine-4(5H)-one; or
3-(acetyloxy)-5-[2-[[2-(3,4-dimethoxyphenyl)ethyl]methylamino]propyl]-2,3-dihydro-2-(4-methoxyphenyl)-1,5-benzothiazepine-4(5H)-one.

10. A process according to any one of claims 1 to 5 wherein said benzothiazepine has the following formula (VI):

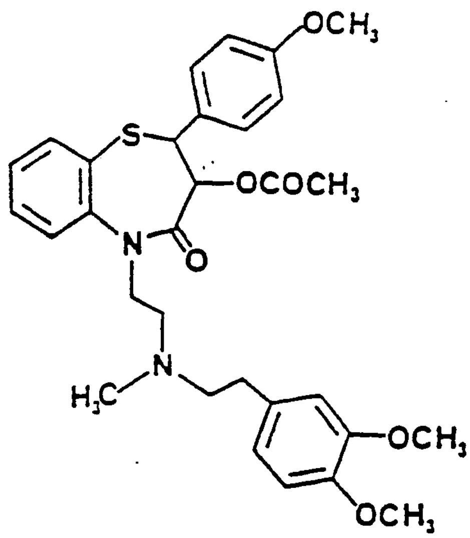

(VI)

or the following formula (VII)

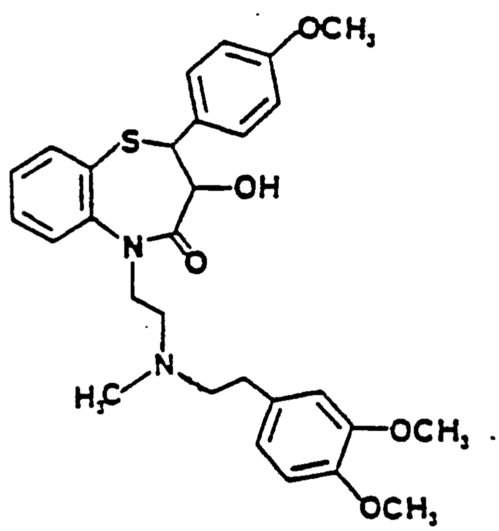

(VII)

11. A process according to any one of claims 1 to 5 wherein said benzothiazepine is the cis isomer.

**Revendications**

**Revendications pour les Etats contractants suivants : BE, CH, DE, FR, GB, IT, LI, LU, NL, SE**

1. Benzothiazépine de la formule suivante (I)

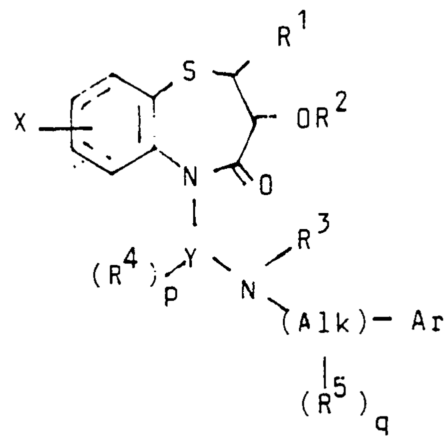

dans laquelle

X est un atome d'hydrogène ou un atome d'halogène;

$R^1$ est phényle ou phényle indépendamment substitué par 1 à 3 parmi méthyle, éthyle, n-propyle, isopropyle, n-butyle, butyle secondaire, butyle tertiaire, iso-butyle, méthoxy, n-propoxy, iso-propoxy, n-butoxy, butoxy secondaire, butoxy tertiaire, iso-butoxy, ou halogène;

$R^2$ est hydrogène, acétyle, propionyle, 2-méthyl-propionyle ou butyryle;

Y est alkylène à chaîne droite de 2 ou 3 carbones;

$R^3$ est méthyle, éthyle, n-propyle, iso-propyle, n-butyle, butyle secondaire ou iso-butyle;

$R^4$ est indépendamment méthyle, éthyle, n-propyle, iso-propyle, n-butyle, butyle secondaire ou iso-butyle;

p est 0, 1, 2 ou 3;

Alk est éthylène, triméthylène ou tétraméthylène;

$R^5$ est hydroxy, alkyle de 1 à 4 carbones ou phényle;

q est 0, 1, 2 ou 3; et

Ar est phényle, phénoxy, thiophénoxy, naphtyle ou un noyau aromatique hétérocyclique à 5 ou 6 membres ayant un atome d'azote, de soufre ou d'oxygène, lesquels noyaux peuvent être substitués indépendamment par un ou plusieurs parmi alkyle, alcoxy ou alkylthio de 1 à 6 carbones chacun, hydroxy, halogène, fluoroalkyle de 1 à 6 carbones, nitro, amino, dialkylamino de 2 à 12 carbones ou méthylènedioxy aux carbones adjacents du noyau,

et les sels d'addition d'acide acceptables en pharmacie, ainsi que les composés d'ammonium quaternaire.

2. Benzothiazépine de la revendication 1, où X est hydrogène.

3. Benzothiazépine de la revendication 1 ou 2, où Alk est éthylène.

4. Benzothiazépine selon l'une des revendications 1 à 3, où :

X est hydrogène;

$R^1$ est phényle substitué par alcoxy $C_{1-4}$;

$R^2$ est alcanoyloxy ayant jusqu'à 4 atomes de carbone;

Y est éthylène;

$R^3$ est alkyle $C_{1-4}$;

p est 0;

Alk est éthylène;

q est 0; et

Ar est phényle ou phényle substitué indépendamment par 1 ou 2 d'alkyle $C_{1-6}$, alcoxy $C_{1-6}$, alkylthio $C_{1-6}$, hydroxy, halogène, fluoro-alkyle $C_{1-6}$, nitro, amino ou dialkylamino $C_{2-12}$ ou par méthylène dioxy aux carbones adjacents du noyau.

5. Benzothiazépine de la revendication 1, où ladite benzothiazépine est :

3-hydroxy-5-[2-[[2-(3-méthoxyphényl)éthyl]méthylamino]éthyl]-2,3-dihydro-2-(4-méthoxyphényl)-1,5-benzothiazépine-4(5H)-one;

3-(hydroxy)-5-[2-[[3,4-dichlorophényl)éthyl]méthylamino]éthyl]-2,3-dihydro-2-(4-méthoxyphényl)-1,5-benzothiazépine-4(5H)-one;

3-(acétyloxy)-5-[2-[[2-(3,4-méthylènedioxyphényl)éthyl]méthylamino]éthyl]-2,3-dihydro-2-(4-méthoxyphényl)-1,5-benzothiazépine-4(5H)-one;

3-hydroxy(5-[2-[[2-(3,4-méthylènedioxyphényl)éthyl]méthylamino]éthyl]-2,3-dihydro-2-(4-méthoxyphényl)-1,5-benzothiazépine-4(5H)-one;

3-(acétyloxy)-5-[2-[[2-(3-méthylphényl)éthyl]méthylamino]éthyl]-2,3-dihydro-2-(4-méthoxyphényl)-1,5-benzothiazépine-4(5H)-one;

3-(acétyloxy)-5-[2-[[2-méthoxyphényl)éthyl]méthylamino]éthyl]-2,3-dihydro-2-(4-méthoxyphényl)1,5-benzothiazépine-4(5H)-one;

3-(acétyloxy)-5-[(S)-2-[[2-(3,4-diméthoxyphényl)éthyl]méthylamino]-1-méthyléthyl]-2,3-dihydro-2-(4-méthoxyphényl)-1,5-benzothiazépin-4(5H)-one;

3-(acétoxy)-5-[(S)-2-[[2-(2,3-dichlorophényl)éthyl]méthylamino]propyl]-2,3-dihydro-2-(4-méthoxyphényl)-1,5-benzothiazépin-4(5H)-one;

3-(acétyloxy)-5-[2-[[2-(3,4-diméthoxyphényl)éthyl]méthylamino]éthyl]-2,3-dihydro-2-(4-méthoxyphényl)-1,5-benzothiazépine-4(5H)-one;

3-(acétyloxy)-5-[2-[[2-(3,5-diméthoxyphényl)éthyl]méthylamino]éthyl]-2,3-dihydro-2-(3-méthoxyphényl)-1,5-benzothiazépine-4(5H)-one;

3-(acétyloxy)-5-[2-[[2-(3,4-dichlorophényl)éthyl]méthylamino]éthyl]-2,3-dihydro-2-(4-méthoxyphényl)-1,5-benzothiazépine-4(5H)-one;

3-(acétyloxy)-5-[2-[[2-(3,4-dichlorophényl)éthyl]méthylamino]éthyl]-2,3-dihydro-2-(4-méthoxyphényl)-1,5-benzothiazépine-4(5H)-one;

3-(acétyloxy)-5-[2-[[2-(3-méthoxyphényl)éthyl]méthylamino]éthyl]-2,3-dihydro-2-(4-méthoxyphényl)-1,5-benzothiazépine-4(5H)-one;

3-(acétyloxy)-5[2-[[2-( 3- diméthylaminophényl)éthyl]méthylamino]éthyl]-2,3-dihydro-2-(4-méthoxyphényl)-1,5-benzothiazépine-4(5H)-one;

3-(acétyloxy)-5-[2-[[2-(3-trifluorométhylphényl)éthyl]méthylamino]éthyl]-2,3-dihydro-2-(4-méthoxyphényl)-1,5-benzothiazépine-4(5H)-one;

3-(acétyloxy)-5-[2-[[2-(3,4-diméthoxyphényl)propyl]méthylamino]éthyl]-2,3-dihydro-2-(4-méthoxyphényl)-1,5-benzothiazépine-4(5H)-one;

5-[2-[[2-(3,4-diméthoxyphényl)éthyl]méthylamino]éthyl]-2,3-dihydro-3-hydroxy-2-(4-méthoxyphényl)-1,5-benzothiazépine-4(5H)-one; ou

3-(acétyloxy)-5-[2-[[2-(3,4-diméthoxyphényl)éthyl]méthylamino]propyl]-2,3-dihydro-2-(4-méthoxyphényl)-1,5-benzothiazépine-4(5H)-one.

**6.** Composé de la formule (VI) qui suit :

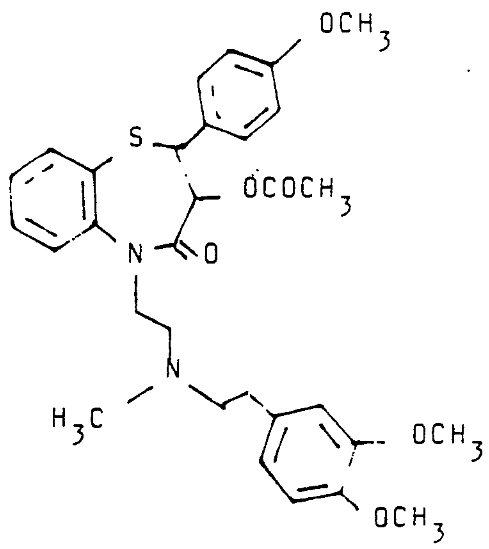

(VI)

ou de la formule (VII)

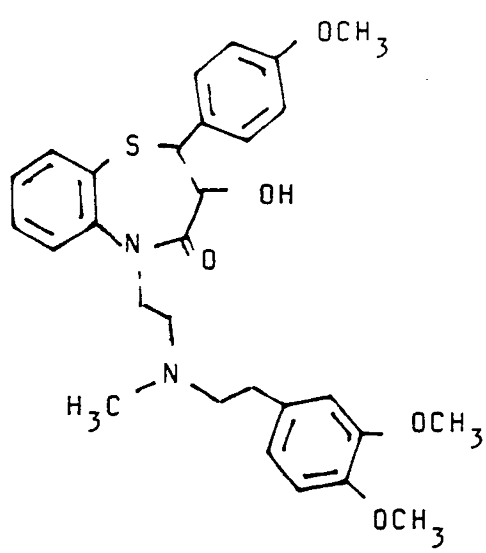

ou son sel d'addition d'acide acceptable en pharmacie.

7. Benzothiazépine selon l'une quelconque des revendications 1 à 6, où ladite benzothiazépine est l'isomère cis.

8. Composition pharmaceutique comprenant une benzothiazépine selon l'une quelconque des revendications 1 à 7 en combinaison avec un diluant ou véhicule acceptable en pharmacie.

9. Benzothiazépine selon l'une quelconque des revendications 1 à 7, ou la composition pharmaceutique de la revendication 8 pour une utilisation dans le traitement de l'angine de poitrine ou de l'hypertension, ou pour la prévention de la récidive des crises cardiaques, chez un mammifère.

**Revendications pour les Etats contractants suivants : AT, ES, GR**

1. Procédé pour la préparation d'une benzothiazépine de la formule suivante (I)

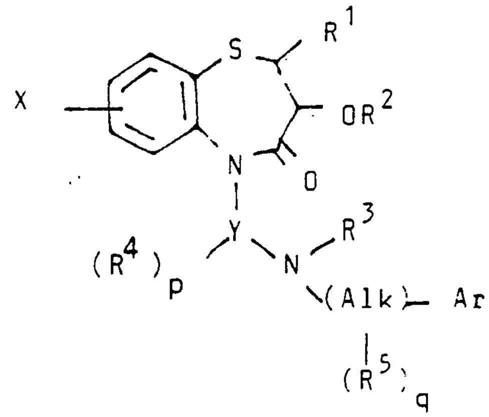

dans laquelle

X est un atome d'hydrogène ou un atome d'halogène;

$R^1$ est phényle ou phényle indépendamment substitué par 1 à 3 parmi méthyle, éthyle, n-propyle, isopropyle, n-butyle, butyle secondaire, butyle tertiaire, iso-butyle, méthoxy, n-propoxy, iso-propoxy, n-butoxy, butoxy secondaire, butoxy tertiaire, iso-butoxy, ou halogène;

$R^2$ est hydrogène, acétyle, propionyle, 2-méthyl-propionyle, ou butyryle;

Y est alkylène à chaîne droite de 2 ou 3 carbones;

$R^3$ est méthyle, éthyle, n-propyle, iso-propyle, n-butyle, butyle secondaire ou iso-butyle;

$R^4$ est indépendamment méthyle, éthyle, n-propyle, iso-propyle, n-butyle, butyle secondaire ou iso-butyle;

p est 0, 1, 2 ou 3;

Alk est éthylène, triméthylène ou tétraméthylène;

$R^5$ est hydroxy, alkyle de 1 à 4 carbones ou phényle;

q est 0, 1, 2 ou 3; et

Ar est phényle, phénoxy, thiophénoxy, naphtyle ou un noyau aromatique hétérocyclique à 5 ou 6 membres ayant un atome d'azote, de soufre ou d'oxygène, lesquels noyaux peuvent être substitués indépendamment par un ou plusieurs parmi alkyle, alcoxy ou alkylthio de 1 à 6 carbones chacun, hydroxy, halogène, fluoroalkyle de 1 à 6 carbones, nitro, amino, dialkylamino de 2 à 12 carbones ou méthylènedioxy aux carbones adjacents du noyau,

et les sels d'addition d'acide acceptables en pharmacie, ainsi que les composés d'ammonium quaternaire,

comprenant :

la réaction d'un composé de la formule suivante (A)

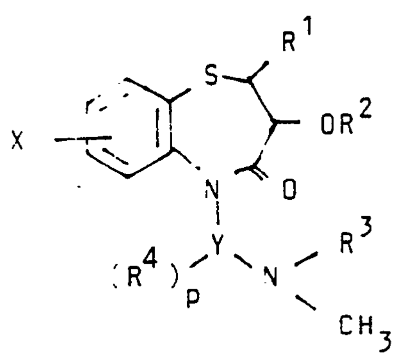
(A)

dans laquelle

X, Y, $R^1$, $R^2$, $R^3$, $R^4$ et p sont tels que définis ci-dessus pour la formule (I), avec un composé de la formule (B) qui suit

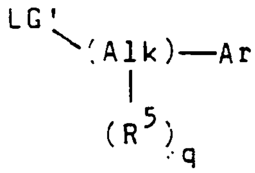

(B)

dans laquelle
$R^5$, q, Alk et Ar sont tels que définis ci-dessus pour la formule (I); et $LG^1$ est un groupe partant approprié, à peu près à température ambiante jusqu'à 50 °C, pour former un conjugué d'un sel d'ammonium quaternaire, puis la réaction du conjugué avec un nucléophile fort et, facultativement, la conversion du produit en un sel d'acide acceptable en pharmacie ou son composé d'ammonium quaternaire.

**2.** Procédé selon la revendication 1, où le groupe partant est tosyle, iodo, mésyle ou bromo.

**3.** Procédé pour la préparation d'une benzothiazépine de la formule (I), telle que définie à la revendication 1, et ses sels d'addition d'acide acceptables en pharmacie et composés d'ammonium quaternaire, comprenant :
la réaction d'un composé de la formule (C) qui suit

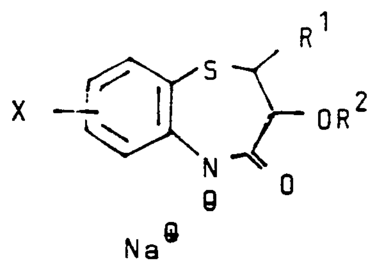

(C)

dans laquelle
X, $R^1$ et $R^2$ sont tels que définis à la revendication 1 pour la formule (I),
avec un composé de la formule qui suit (D)

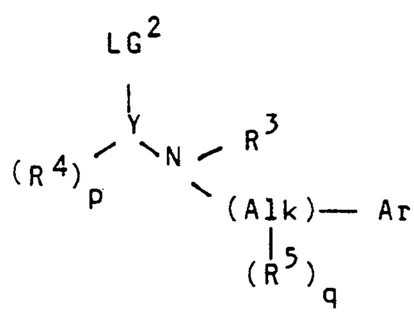

(D)

où
$R^3$, $R^4$, $R^5$, p, q, Alk, Ar et Y sont tels que définis à la revendication 1 pour la formule (I); et $LG^2$ est un groupe partant approprié, à une température d'environ 25 à 150 °C et, facultativement, la conversion du produit en un sel d'addition d'acide acceptable en pharmacie ou un composé d'ammonium quaternaire.

**4.** Procédé selon la revendication 3, où le groupe partant est chlore ou brome.

**5.** Procédé pour la préparation d'une composition pharmaceutique comprenant le mélange d'une quantité pharmaceutiquement efficace d'une benzothiozépine de la formule (I), telle que définie à la revendication 1, ou son sel d'addition d'acide acceptable en pharmacie ou son composé d'ammonium quaternaire avec un diluant, véhicule ou excipient acceptables en pharmacie.

6. Procédé selon l'une quelconque des revendications 1 à 5, où X est hydrogène.

7. Procédé selon l'une quelconque des revendications 1 à 6, où Alk est éthylène.

8. Procédé selon l'une quelconque des revendications 1 à 7, où
X est hydrogène;
$R^1$ est phényle substitué par alcoxy $C_1$-$C_4$;
$R^2$ est alcanoyloxy ayant jusqu 'à 4 atomes de carbone;
Y est éthylène;
$R^3$ est alkyle $C_{1-4}$.
p est 0;
Alk est éthylène;
q est 0; et
Ar est phényle ou phényle indépendamment substitué par 1 ou 2 parmi alkyle $C_{1-6}$, alcoxy $C_{1-6}$, alkylthio $C_{1-6}$, hydroxy, halogène, fluoro alkyle $C_{1-6}$, nitro, amino ou dialkylamino $C_{1-12}$ ou par méthylène dioxy aux carbones adjacents du noyau.

9. Procédé selon l'une quelconque des revendications 1 à 5, où ladite benzothiazépine est :

3-(hydroxy)-5-[2-[[2-(3-méthoxyphényl)éthyl]méthylamino]éthyl]-2,3-dihydro-2-(4-méthoxyphényl)-1,5-benzothiazépine-4(5H)-one;

3-(hydroxy)-5-[2-[[3.--(dichlorophényl)éthyl]méthylamino]éthyl]-2,3-dihydro-2-(4-méthoxyphényl)-1,5-benzothiazépine-4(5H)-one;

3-(acétyloxy)-5-[2-[[2-(3,4-méthylènedioxyphényl)éthyl]méthylamino]éthyl]-2,3-dihydro-2-(4-méthoxyphényl)-1,5-benzothiazépine-4(5H)-one;

3-hydroxy(5-[2-[[2-(3.--méthylènedioxyphényl)éthyl]méthylamino]éthyl]-2,3-dihydro-2-(4-méthoxyphényl)-1,5-benzothiazépine-4(5H)-one;

3-(acétyloxy)-5-[2-[[2-(3-méthylphényl)éthyl]méthylamino]éthyl]-2,3-dihydro-2-(4-méthoxyphényl)-1,5-benzothiazépine -4(5H)-one;

3-(acétyloxy)-5-[2-[[2-méthoxyphényl)éthyl]méthylamino]éthyl]-2,3-dihydro-2-(4-méthoxyphényl)1,5-benzothiazépine-4(5H)-one;

3-(acétyloxy)-5-[(S)-2-[[2-(3,4-diméthoxyphényl)éthyl]méthylamino]-1-méthyléthyl]-2,3-dihydro-2-(4-méthoxyphényl)-1,5-benzothiazépin-4(5H)one;

3-(acétoxy)-5-[(S)-2-[[2-(2,3-dichlorophényl)éthyl]méthylamino]propyl]-2,3-dihydro-2-(4-méthoxyphényl)-1,5-benzothiazépin-4(5H)-one;

3-(acétyloxy)-5-[2-[[2-(3,4-diméthoxyphényl)éthyl]méthylamino]éthyl]-2,3-dihydro-2-(4-méthoxyphényl)-1,5-benzothiazépine-4(5H)-one;

3-(acétyloxy)-5-[2-[[2-(3,4-diméthoxyphényl)éthyl]méthylamino]éthyl]-2,3-dihydro-2-(3-méthoxyphényl)-1,5-benzothiazépine-4(5H)-one;

3-(acétyloxy)-5-[2-[[2-(3,4-dichlorophényl)éthyl]méthylamino]éthyl]-2,3-dihydro-2-(4-méthoxyphényl)-1,5-benzothiazépine-4(5H)-one;

3-(acétyloxy)-5-[2-[[2-(3,4-dichlorophényl)éthyl]méthylamino]éthyl]-2,3-dihydro-2-(4-méthoxyphényl)-1,5-benzothiazépine-4(5H)-one;

3-(acétyloxy)-5-[2-[[2-(3-méthoxyphényl)éthyl]méthylamino]éthyl]-2,3-dihydro-2-(4-méthoxyphényl)-1,5-benzothiazépine-4(5H)-one;

3-(acétyloxy)-5[2-[[2-( 3- diméthylaminophényl)éthyl]méthylamino]éthyl]-2,3-dihydro-2-(4-méthoxyphényl)-1,5-benzothiazépine-4(5H)-one;

3-(acétyloxy)-5-[2-[[2-(3-trifluorométhylphényl)éthyl]méthylamino]éthyl]-2,3-dihydro-2-(4-méthoxyphényl)-1,5-benzothiazépine-4(5H)-one;

3-(acétyloxy)-5-[2-[[2-(3,4-diméthoxyphényl)propyl]méthylamino]éthyl]-2,3-dihydro-2-(4-méthoxyphényl)-1,5-benzothiazépine-4(5H)-one;

5-[2-[[2-(3,4-diméthoxyphényl)éthyl]méthylamino]éthyl]-2,3-dihydro-3-hydroxy-2-(4-méthoxyphényl)-1,5-benzothiazépine-4(5H)-one; ou

3-(acétyloxy)-5-[2-[[2-(3,4-diméthoxyphényl)éthyl]méthylamino]propyl]-2,3-dihydro-2-(4-méthoxyphényl)-1,5-benzothiazépine-4(5H)-one.

**10.** Procédé selon l'une quelconque des revendications 1 à 5, où ladite benzothiazépine a la formule (VI) qui suit :

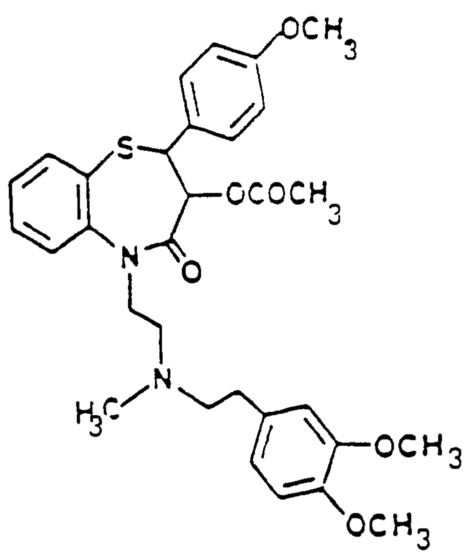

(VI)

ou la formule qui suit (VII)

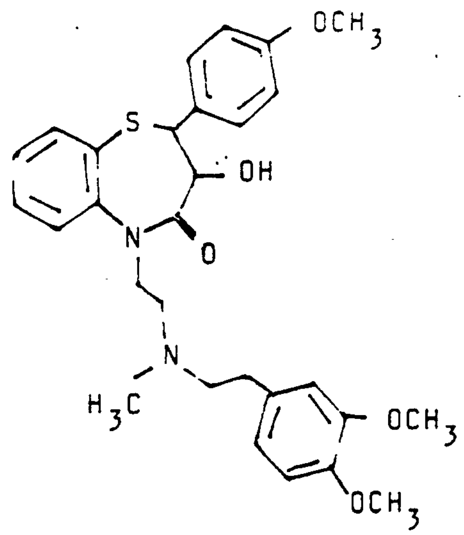

(VII)

11. Procédé selon l'une quelconque des revendications 1 à 5, où ladite benzothiazépine est l'isomère cis.

**Ansprüche**
**Patentansprüche für folgende Vertragsstaaten : BE, CH, DE, FR, GB, IT, LI, LU, NL, SE**

1. Ein Benzothiazepin der nachstehenden Formel (I):

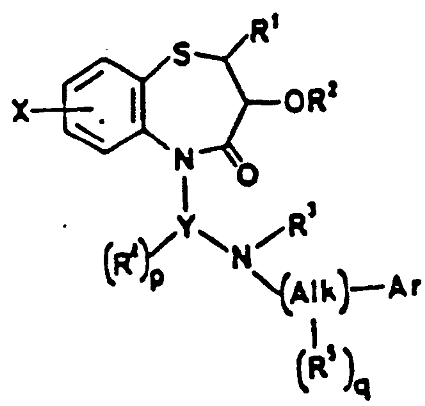

,

worin:

| | |
|---|---|
| X | ein Wasserstoffatom oder ein Halogenatom darstellt; |
| $R^1$ | für Phenyl steht oder für ein unabhängig voneinander durch 1 bis 3 Reste aus Methyl, Ethyl, n-Propyl, Isopropyl, n-Butyl, sek.Butyl, tert.Butyl, iso-Butyl, Methoxy, Ethoxy, n-Propoxy, iso-Propoxy, n-Butoxy, sek.Butoxy, tert.Butoxy, iso-Butoxy oder Halogen substituiertes Phenyl steht; |
| $R^2$ | für Wasserstoff Acetyl, Propionyl, 2-Methylpropionyl oder Butyryl steht; |
| Y | eine gerade Alkylenkette mit 2 oder 3 Kohlenstoffatomen darstellt; |
| $R^3$ | für Methyl, Ethyl, n-Propyl, iso-Propyl, n-Butyl, sek.Butyl oder iso-Butyl steht; |
| $R^4$ | unabhängig voneinander Methyl, Ethyl, n-Propyl, iso-Propyl, n-Butyl, sek.Butyl oder iso-Butyl bedeutet; |
| p | den Wert 0, 1, 2 oder 3 aufweist; |
| Alk | für Ethylen, Trimethylen oder Tetramethylen steht; |
| $R^5$ | für Hydroxy, Alkyl mit 1 bis 4 Kohlenstoffatomen oder Phenyl steht; |
| q | den Wert 0, 1, 2 oder 3 aufweist; und |
| Ar | für Phenyl, Phenoxy, Thiophenoxy, Naphthyl oder einen 5- oder 6-gliedrigen heterocycli- |

schen aromatischen Ring mit einem Stickstoff-, Schwefel- oder Sauerstoffatom steht, welche Ringe unabhängig voneinander durch einen oder mehrere Alkyl-, Alkoxy- oder Alkylthioreste mit jeweils 1 bis 6 Kohlenstoffatomen, Hydroxyreste, Halogenreste, Fluoralkylreste mit 1 bis 6 Kohlenstoffatomen, Nitroreste, Aminoreste, Dialkylaminoreste mit 2 bis 12 Kohlenstoffatomen oder Methylendioxyreste an benachbarten Ringkohlenstoffatomen substituiert sein können,

und die pharmazeutisch annehmbaren Säureadditionssalze und quaternären Ammoniumverbindungen hievon.

2. Benzothiazepin nach Anspruch 1, worin X für Wasserstoff steht.

3. Benzothiazepin nach Anspruch 1 oder 2, worin Alk für Ethylen steht.

4. Benzothiazepin nach einem der Ansprüche 1 bis 3, worin:
   - X für Wasserstoff steht;
   - $R^1$ für $C_{1-4}$-Alkoxy-substituiertes Phenyl steht;
   - $R^2$ für Alkanoyloxy mit bis zu 4 Kohlenstoffatomen steht;
   - Y Ethylen bedeutet;
   - $R^3$ für $C_{1-4}$-Alkyl steht;
   - p den Wert 0 hat;
   - Alk für Ethylen steht;
   - q den Wert 0 hat; und
   - Ar für Phenyl oder substituiertes Phenyl steht, das unabhängig voneinander durch 1 oder 2 Reste aus $C_{1-6}$-Alkyl, $C_{1-6}$-Alkoxy, $C_{1-6}$-Alkylthio, Hydroxy, Halogen, Fluor-$C_{1-6}$-alkyl, Nitro, Amino oder $C_{2-12}$-Dialkylamino oder an benachbarten Ringkohlenstoffatomen durch Methylendioxy substituiert ist.

5. Benzothiazepin nach Anspruch 1, worin das Benzothiazepin eine der folgenden Verbindungen ist:
3-Hydroxy-5-[2-[[2-(3-methoxyphenyl)ethyl]methylamino]ethyl]-2,3-dihydro-2-(4-methoxyphenyl)-1,5-benzothiazepin-4(5H)-on;
3-(Hydroxy)-5-[2-[[2-(3,4-dichlorphenyl)ethyl]methylamino]ethyl]-2,3-dihydro-2-(4-methoxyphenyl)-1,5-benzothiazepin-4(5H)-on;
3-(Acetyloxy)-5-[2-[[2-(3,4-methylendioxyphenyl)ethyl]methylamino]ethyl]-2,3-dihydro-2-(4-methoxyphenyl)-1,5-benzothiazepin-4(5H)-on;
3-Hydroxy-5-[2-[[2-(3,4-Methylendioxyphenyl)ethyl]methylamino]ethyl]-2,3-dihydro-2-(4-methoxyphenyl)-1,5-benzothiazepin-4(5H)-on;
3-(Acetyloxy)-5-[2-[[2-(3-methylphenyl)ethyl]methylamino]ethyl]-2,3-dihydro-2-(4-methoxyphenyl)-1,5-benzothiazepin-4(5H)-on;
3-(Acetyloxy)-5-[2-[[2-methoxyphenyl)ethyl]methylamino]ethyl]-2,3-dihydro-2-(4-methoxyphenyl)-1,5-benzothiazepin-4(5H)-on;
3-(Acetyloxy)-5-[(S)-2-[[2-(3,4-dimethoxyphenyl)ethyl]methylamino)-1-methylethyl]-2,3-dihydro-2-(4-methoxyphenyl)-1,5-benzothiazepin-4(5H)-on;
3-(Acetoxy)-5-[(S)-2-[[2-(2,3-dichlorphenyl)ethyl]methylamino]propyl]-2,3-dihydro-2-(4-methoxyphenyl)-1,5-benzothiazepin-4(5H)-on;
3-(Acetyloxy)-5-[2-[[2-(3,4-dimethoxyphenyl)ethyl]methylamino]ethyl]-2,3-dihydro-2-(4-methoxyphenyl)-1,5-benzothiazepin-4(5H)-on;
3-Acetyloxy)-5-[2-[[2-(3,5-dimethoxyphenyl)ethyl]methylamino]ethyl]-2,3-dihydro-2-(4-methoxyphenyl)-1,5-benzothiazepin(-4(5H)-on;
3-(Acetyloxy)-5-[2-[[2-(3,4-dichlorphenyl)ethyl]methylamino]ethyl]-2,3-dihydro-2-(4-methoxyphenyl)-1,5-benzothiazepin-4(5H)-on;
3-(Acetyloxy)-5-[2-[[2-(2,3-dichlorphenyl)ethyl]methylamino]ethyl]-2,3-dihydro-2-(4-methoxyphenyl)-1,5-benzothiazepin-4(5H)-on;
3-(Acetyloxy)-5-[2-[[2-(3-methoxyphenyl)ethyl]methylamino]ethyl]-2,3-dihydro-2-(4-methoxyphenyl)-1,5-benzothiazepin-4(5H)-on;
3-(Acetyloxy)-5-[2-[[2-(3-dimethylaminophenyl)ethyl]methylamino]ethyl]-2,3-dihydro-2-(4-methoxyphenyl)-1,5-benzothiazepin-4(5H)-on;
3-(Acetyloxy)-5-[2-[[2-(3-trifluormethylphenyl)ethyl]methylamino]ethyl]-2,3-dihydro-2-(4-methoxyphenyl)-1,5-benzothiazepin-4(5H)-on;

3-(Acetyloxy)-5-[2-[[2-(3,4-dimethoxyphenyl)propyl]methylamino]ethyl]-2,3-dihydro-2-(4-methoxyphenyl)-1,5-benzothiazepin-4(5H)-on;
5-[2-[[2-(3,4-Dimethoxyphenyl)ethyl]methylamino]ethyl]-2,3-dihydro-3-hydroxy-2-(4-methoxyphenyl)-1,5-benzothiazepin-4(5H)-on; oder
3-(Acetyloxy)-5-[2-[[2-(3,4-dimethoxyphenyl)ethyl]methylamino]propyl]-2,3-dihydro-2-(4-methoxyphenyl)-1,5-benzothiazepin-4(5H)-on.

6. Verbindung der nachfolgenden Formel (VI):

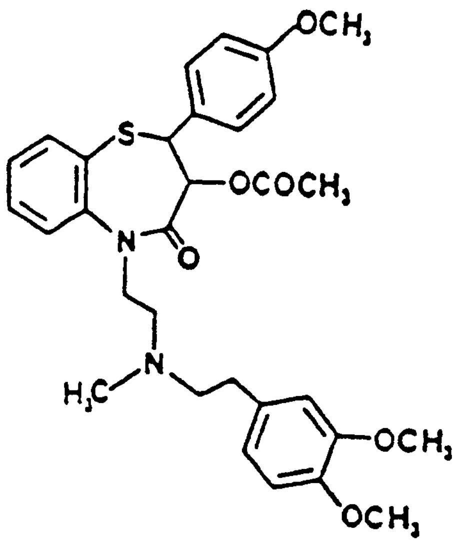

(VI)

oder der Formel (VII):

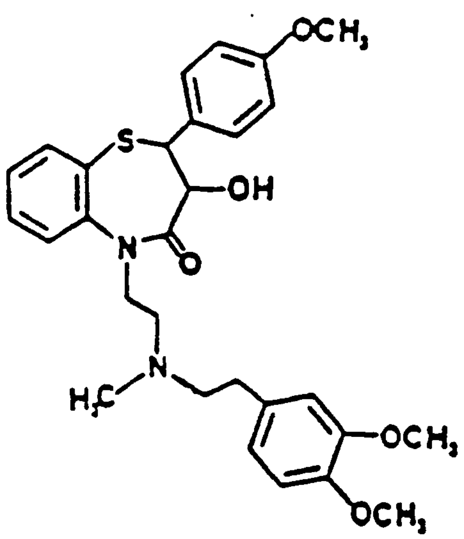

(VII)

oder ein pharmazeutisch annehmbares Säureadditionssalz hievon.

7. Benzothiazepin nach einem der Ansprüche 1 bis 6, worin das Benzothiazepin das cis-Isomer ist.

8. Pharmazeutische Zusammensetzung, umfassend ein Benzothiazepin nach einem der Ansprüche 1 bis 7 in Kombination mit einem pharmazeutisch annehmbaren Verdünnungsmittel oder Träger.

9. Das Benzothiazepin nach einem der Ansprüche 1 bis 7 oder die pharmazeutische Zusammensetzung nach Anspruch 8 zur Anwendung in der Behandlung von Angina Pectoris oder Bluthochdruck, oder zur

Verhütung des Wiederauftretens von Herzanfällen in einem Säugetier.

**Patentansprüche für folgende Vertragsstaaten : AT, ES, GR**

1. Verfahren zur Herstellung eines Benzothiazepins der nachstehenden Formel (I):

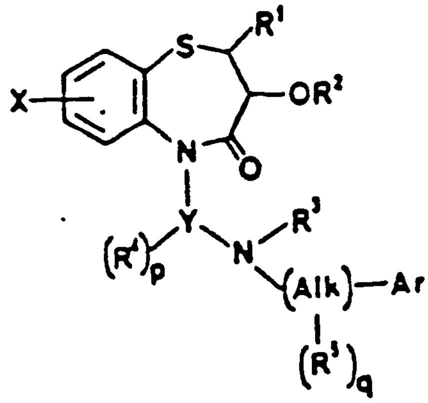

(I)

worin:

X ein Wasserstoffatom oder ein Halogenatom darstellt;

$R^1$ für Phenyl steht oder für ein unabhängig voneinander durch 1 bis 3 Reste aus Methyl, Ethyl, n-Propyl, Isopropyl, n-Butyl, sek.Butyl, tert.Butyl, iso-Butyl, Methoxy, Ethoxy, n-Propoxy, iso-Propoxy, n-Butoxy, sek.Butoxy, tert.Butoxy, iso-Butoxy oder Halogen substituiertes Phenyl steht;

$R^2$ für Wasserstoff, Acetyl, Propionyl, 2-Methylpropionyl oder Butyryl steht;

Y eine gerade Alkylenkette mit 2 oder 3 Kohlenstoffatomen darstellt;

$R^3$ für Methyl, Ethyl, n-Propyl, iso-Propyl, n-Butyl, sek.Butyl oder iso-Butyl steht;

$R^4$ unabhängig voneinander Methyl, Ethyl, n-Propyl, iso-Propyl, n-Butyl, sek.Butyl oder iso-Butyl bedeutet;

p den Wert 0, 1, 2 oder 3 aufweist;

Alk für Ethylen, Trimethylen oder Tetramethylen steht;

$R^5$ für Hydroxy, Alkyl mit 1 bis 4 Kohlenstoffatomen oder Phenyl steht;

q den Wert 0, 1, 2 oder 3 aufweist; und

Ar für Phenyl, Phenoxy, Thiophenoxy, Naphthyl oder einen 5- oder 6-gliedrigen heterocyclischen aromatischen Ring mit einem Stickstoff-, Schwefel- oder Sauerstoffatom steht, welche Ringe unabhängig voneinander durch einen oder mehrere Alkyl-, Alkoxy- oder Alkylthioreste mit jeweils 1 bis 6 Kohlenstoffatomen, Hydroxyreste, Halogenreste, Fluoralkylreste mit 1 bis 6 Kohlenstoffatomen, Nitroreste, Aminoreste, Dialkylaminoreste mit 2 bis 12 Kohlenstoffatomen oder Methylendioxyreste an benachbarten Ringkohlenstoffatomen substituiert sein können,

und den pharmazeutisch annehmbaren Säureadditionssalzen und quaternären Ammoniumverbindungen hievon, umfassend ein Umsetzen einer Verbindung der nachstehenden Formel (A):

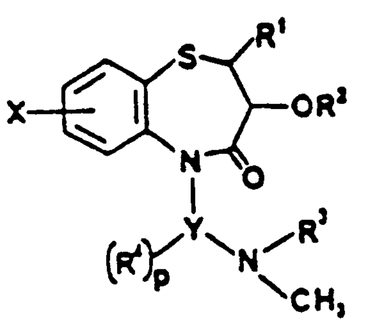

(A) ,

worin
X, Y, $R^1$, $R^2$, $R^3$, $R^4$ und p wie oben für Formel (I) definiert sind, mit einer Verbindung der folgenden Formel (B):

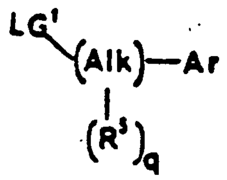

(B) ,

worin
$R^5$, q, Alk und Ar wie oben für Formel (I) definiert sind; und $LG^1$ eine geeignete Leaving-Gruppe bedeutet, bei einer Temperatur von etwa Raumtemperatur bis zu 150° C, zur Ausbildung eines quaternären Ammoniumsalz-Konjugats und ein anschließendes Umsetzen des Konjugats mit einem stark nukleophilen Reagens und gewünschtenfalls ein Umwandeln des Produktes in ein pharmazeutisch annehmbares Säureadditionssalz oder eine quaternäre Ammoniumverbindung hievon.

2. Verfahren nach Anspruch 1, worin die Leaving-Gruppe Tosyl, Iodid, Mesyl oder Bromid ist.

3. Verfahren zur Herstellung eines Benzothiazepins der Formel (I), wie in Anspruch 1 definiert, und der pharmazeutisch annehmbaren Säureadditionssalze und quaternären Ammoniumverbindungen hievon, umfassend ein Umsetzen einer Verbindung der nachstehenden Formel (C):

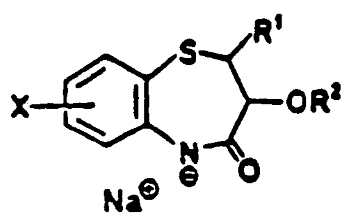

(C) ,

worin
X, $R^1$ und $R^2$ wie in Anspruch 1 für Formel (I) definiert sind, mit einer Verbindung der folgenden Formel (D):

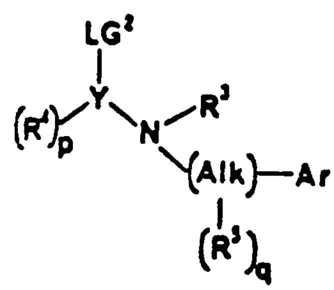

(D) ,

worin
$R^3$, $R^4$, $R^5$, p, q, Alk, Ar und Y wie in Anspruch 1 für Formel (I) definiert sind; und $LG^2$ eine geeignete Leaving-Gruppe bedeutet, bei einer Temperatur von etwa 25 bis 150° C, und gewünschtenfalls ein Umwandeln des Produktes in ein pharmazeutisch annehmbares Säureadditionssalz oder eine quaternäre Ammoniumverbindung hievon.

4. Verfahren nach Anspruch 3, worin die Leaving-Gruppe Chlor oder Brom ist.

5. Verfahren zur Herstellung einer pharmazeutischen Zusammensetzung, umfassend ein Vermischen einer pharmazeutisch wirksamen Menge eines Benzothiazepins der Formel (I), wie in Anspruch 1 definiert, oder eines pharmazeutisch annehmbaren Säureadditionssalzes oder einer quaternären Ammoniumverbindung hievon mit einem pharmazeutisch annehmbaren Verdünnungsmittel, Träger oder Exzipiens.

6. Verfahren nach einem der Ansprüche 1 bis 5, worin X für Wasserstoff steht.

7. Verfahren nach einem der Ansprüche 1 bis 6, worin Alk für Ethylen steht.

8. Verfahren nach einem der Ansprüche 1 bis 7, worin:

| | |
|---|---|
| X | für Wasserstoff steht; |
| $R^1$ | für $C_{1-4}$-Alkoxy-substituiertes Phenyl steht; |
| $R^2$ | für Alkanoyloxy mit bis zu 4 Kohlenstoffatomen steht; |
| Y | Ethylen bedeutet; |
| $R^3$ | für $C_{1-4}$-Alkyl steht; |
| p | den Wert 0 hat; |
| Alk | für Ethylen steht; |
| q | den Wert 0 hat; und |
| Ar | für Phenyl oder substituiertes Phenyl steht, das unabhängig voneinander durch 1 oder 2 Reste aus $C_{1-6}$-Alkyl, $C_{1-6}$-Alkoxy, $C_{1-6}$-Alkylthio, Hydroxy, Halogen, Fluor-$C_{1-6}$-alkyl, Nitro, Amino oder $C_{2-12}$-Dialkylamino oder an benachbarten Ringkohlenstoffatomen durch Methylendioxy substituiert ist. |

9. Verfahren nach einem der Ansprüche 1 bis 5, worin das Benzothiazepin eine der folgenden Verbindungen ist:
3-Hydroxy-5-[2-[[2-(3-methoxyphenyl)ethyl]methylamino]ethyl]-2,3-dihydro-2-(4-methoxyphenyl)-1,5-benzothiazepin-4(5H)-on;
3-(Hydroxy)-5-[2-[[2-(3,4-dichlorphenyl)ethyl]methylamino]ethyl]-2,3-dihydro-2-(4-methoxyphenyl)-1,5-benzothiazepin-4(5H)-on;
3-(Acetyloxy)-5-[2-[[2-(3,4-methylendioxyphenyl)ethyl]methylamino]ethyl]-2,3-dihydro-2-(4-methoxyphenyl)-1,5-benzothiazepin-4(5H)-on;
3-Hydroxy-5-[2-[[2-(3,4-Methylendioxyphenyl)ethyl]methylamino]ethyl]-2,3-dihydro-2-(4-methoxyphenyl)-1,5-benzothiazepin-4(5H)-on;
3-(Acetyloxy)-5-[2-[[2-(3-methylphenyl)ethyl]methylamino]ethyl]-2,3-dihydro-2-(4-methoxyphenyl)-1,5-benzothiazepin-4(5H)-on;
3-(Acetyloxy)-5-[2-[[2-methoxyphenyl)ethyl]methylamino]ethyl]-2,3-dihydro-2-(4-methoxyphenyl)-1,5-benzothiazepin-4(5H)-on;
3-(Acetyloxy)-5-[(S)-2-[[2-(3,4-dimethoxyphenyl)ethyl]methylamino]-1-methylethyl]-2,3-(dihydro-2-(4-methoxyphenyl)-1,5-benzothiazepin-4(5H)-on;
3-(Acetoxy)-5-[(S)-2-[[2-(2,3-dichlorphenyl)ethyl]methylamino]propyl]-2,3-dihydro-2-(4-methoxyphenyl)-1,5-benzothiazepin-4(5H)-on;
3-(Acetyloxy)-5-[2-[[2-(3,4-dimethoxyphenyl)ethyl]methylamino]ethyl]-2,3-dihydro-2-(4-methoxyphenyl)-1,5-benzothiazepin-4(5H)-on;
3-Acetyloxy)-5-[2-[[2-(3,5-dimethoxyphenyl)ethyl]methylamino]ethyl]-2,3-dihydro-2-(4-methoxyphenyl)-1,5-benzothiazepin-4(5H)-on;
3-(Acetyloxy)-5-[2-[[2-(3,4-dichlorphenyl)ethyl]methylamino]ethyl]-2,3-dihydro-2-(4-methoxyphenyl)-1,5-benzothiazepin-4(5H)-on;
3-(Acetyloxy)-5-[2-[[2-(2,3-dichlorphenyl)ethyl]methylamino]ethyl]-2,3-dihydro-2-(4-methoxyphenyl)-1,5-benzothiazepin-4(5H)-on;
3-(Acetyloxy)-5-[2-[[2-(3-methoxyphenyl)ethyl]methylamino]ethyl]-2,3-dihydro-2-(4-methoxyphenyl)-1,5-benzothiazepin-4(5H)-on;
3-(Acetyloxy)-5-[2-[[2-(3-dimethylaminophenyl)ethyl]methylamino]ethyl]-2,3-dihydro-2-(4-methoxyphenyl)-1,5-benzothiazepin-4(5H)-on;
3-(Acetyloxy)-5-[2-[[2-(3-trifluormethylphenyl)ethyl]methylamino]ethyl]-2,3-dihydro-2-(4-methoxyphenyl)-1,5-benzothiazepin-4(5H)-on;
3-(Acetyloxy)-5-[2-[[2-(3,4-dimethoxyphenyl)propyl]methylamino]ethyl]-2,3-dihydro-2-(4-methoxyphenyl)-1,5-benzothiazepin-4(5H)-on;
5-[2-[[2-(3,4-Dimethoxyphenyl)ethyl]methylamino]ethyl]-2,3-dihydro-3-hydroxy-2-(4-methoxyphenyl)-1,5-benzothiazepin-4(5H)-on; oder
3-(Acetyloxy)-5-[2-[[2-(3,4-dimethoxyphenyl)ethyl]methylamino]propyl]-2,3-dihydro-2-(4-methoxyphenyl)-1,5-benzothiazepin-4(5H)-on.

10. Verfahren nach einem der Ansprüche 1 bis 5, worin das Benzothiazepin die folgende Formel (VI):

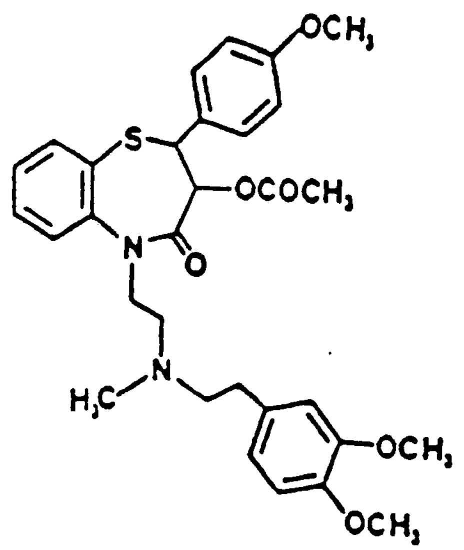

(VI)

oder die folgende Formel (VII):

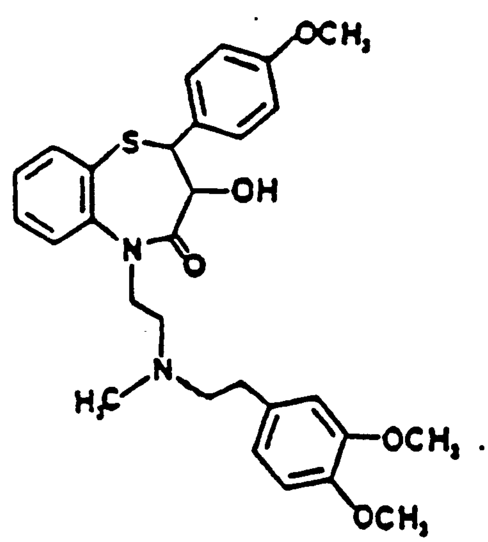

(VII)

aufweist.

11. Verfahren nach einem der Ansprüche 1 bis 5, worin das Benzothiazepin das cis-Isomer ist.